# EUROPEAN PATENT SPECIFICATION

(11) **EP 2 617 283 B1**
(45) Date of publication and mention of the grant of the patent: **22.07.2015**
(21) Application number: 11825288.1
(22) Date of filing: 16.09.2011
(51) Int. Cl.: C12N 15/82, A01H 5/02

(54) **METHOD FOR CULTIVATING LILIES CONTAINING DELPHININ IN THE PETALS THEREOF**
VERFAHREN ZUR KULTIVIERUNG VON LILIEN MIT DELPHININ IN DEREN BLÜTENBLÄTTERN
PROCÉDÉ POUR CULTIVER DES LYS CONTENANT DE LA DELPHINIDINE DANS LEURS PÉTALES

(30) Priority: 17.09.2010 JP 2010209349
(43) Date of publication of application: 24.07.2013
(73) Proprietor: Suntory Holdings Limited, Osaka-shi, Osaka 530-8203 (JP); Niigata Prefecture, Niigata-shi, Niigata 950-8570 (JP)
(72) Inventor: TANAKA, Yoshikazu, Mishima-gun Osaka 618-8503 (JP); NAKAMURA, Noriko, Mishima-gun Osaka 618-8503 (JP); KOBAYASHI, Hitoshi, Nagaoka-shi Niigata 940-0826 (JP); OKUHARA, Hiroaki, Nagaoka-shi Niigata 940-0826 (JP); KONDO, Masayoshi, Nagaoka-shi Niigata 940-0826 (JP); KOIKE, Yosuke, Nagaoka-shi Niigata 940-0826 (JP); HOSHI, Yosuke, Uonuma-shi Niigata 946-0004 (JP); NOMIZU, Toshikazu, Kitakamahara-gun Niigata 957-0111 (JP)
(74) Representative: Keirstead, Tanis Evelyne
(86) International application number: PCT/JP2011/071271
(87) International publication number: WO 2012/036290

(56) References cited:
- WO-A1-2009/062253
- WO-A1-2009/062253
- WO-A1-2010/069004
- JP-A- H08 511 683
- US-A1- 2002 100 072
- YOSHIKAZU TANAKA ET AL: "Genetic engineering in floriculture", PLANT CELL, TISSUE AND ORGAN CULTURE, KLUWER ACADEMIC PUBLISHERS, DO, vol. 80, no. 1, 1 January 2005 (2005-01-01), pages 1-24, XP019268499, ISSN: 1573-5044
- TANAKA YOSHIKAZU ET AL: "Flower Color Modification by Engineering of the Flavonoid Biosynthetic Pathway: Practical Perspectives", BIOSCIENCE BIOTECHNOLOGY AND BIOCHEMISTRY, vol. 74, no. 9, 7 September 2010 (2010-09-07), pages 1760-1769, XP002718419,
- YOSHIKAZU TANAKA: "Flower colour and cytochromes P450", PHYTOCHEMISTRY REVIEWS, KLUWER ACADEMIC PUBLISHERS, DO, vol. 5, no. 2-3, 31 October 2006 (2006-10-31), pages 283-291, XP019453401, ISSN: 1572-980X, DOI: 10.1007/S11101-006-9003-7
- TANAKA YOSHIKAZU ET AL: "CHAPTER 9: Flower colour", FLOWERING AND ITS MANIPULATION (BOOK SERIES: ANNUAL PLANT REVIEWS), BLACKWELL PUBLISHING, vol. 20, 1 January 2006 (2006-01-01), pages 201-239, XP009134887, ISBN: 978-1-4051-2808-7
- TANAKA Y. ET AL.: 'Flower color modification by engineering of the flavonoid biosynthetic pathway: Pratical perspectives' BIOSCI. BIOTECHNOL.BIOCHEM. vol. 74, no. 9, 2010, pages 1760 - 1769, XP002718419
- MARTENS S. ET AL.: 'Impact of biochemical pre-studies on specific metabolic engineering strategies of flavonoid biosynthesis in plant tissues' BIOCHEM.ENGINEER.J. vol. 14, 2003, pages 227 - 235, XP055095748
- SHIRO MORI ET AL.: 'Agrobacterium-ho ni yoru Hototogisu (Tricyrilis hirta) eno Anthocyanin Seigosei Keiro Kanren Idenshi no Donyu' JOURNAL OF THE JAPANESE SOCIETY FOR HORTICULTURAL SCIENCE vol. 74, 1, 2005, page 502
- OKINAKA Y. ET AL.: 'Selective accumulation of delphinidin derivatives in Tabacco using a putative flavonoid 3',5'-hydroxylase cDNA from campanula medium' BIOSCI.BIOTECHNOL.BIOCHEM. vol. 67, no. 1, 2003, pages 161 - 165, XP055095775

## Description

### TECHNICAL FIELD

The present invention relates to a method for producing lilies containing a blue pigment (delphinidin) in the petals thereof by introducing a foreign gene into lilies.

### BACKGROUND ART

Methods have been previously reported for inducing the production of delphinidin in plant cells by introducing a foreign gene in carnations (see Patent Document 1), chrysanthemums (see Patent Document 1), roses (see Patent Document 1 and Patent Document 2), moth orchids (see Patent Document 3) and cymbidium (see Patent Document 3).

However, cases of successfully producing lilies having blue petals by synthesizing delphinidin in lily petals using the action of an introduced foreign gene have yet to be reported.

Examples of methods used to improve plant varieties include (1) crossbreeding by crossing stamen and pistil, (2) spontaneous or artificial mutation, and (3) genetic recombination. The use of genetic recombination technology enables expression a useful gene in a plant without being constrained by genetic restrictions possessed by the target species, thereby making it possible to impart a new trait to a plant that is not inherently possessed by that plant. Genetically modified plants produced by genetic recombination technology are currently cultivated over a wide range throughout the world.

Examples of plant pigment components include anthocyanin, carotenoid and betalain.

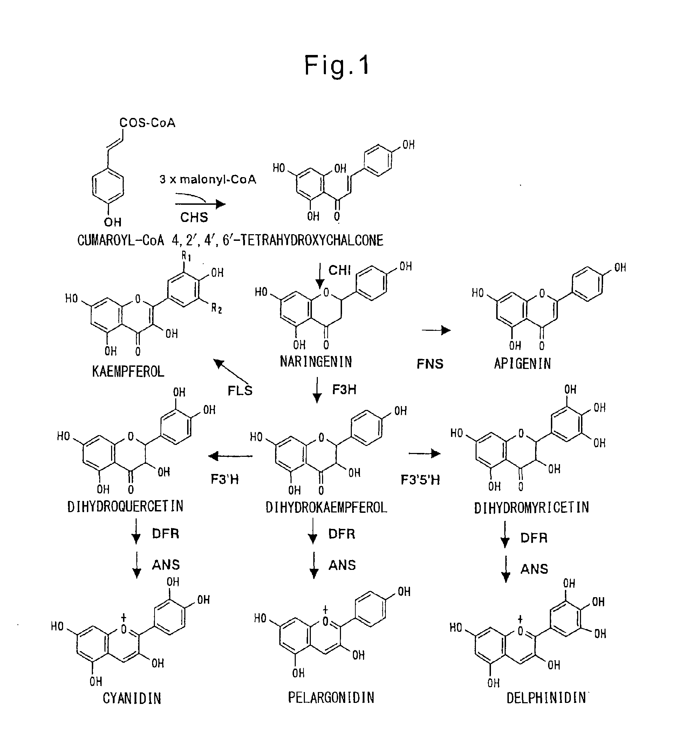

Anthocyanins are members of the group of secondary metabolites referred to as flavonoids, and as shown in FIG. 1, are synthesized by the actions of numerous enzymes extending from phenylalanine to coumaroyl-CoA. The color of anthocyanins is dependent on the structure thereof. Namely, as the number of hydroxyl groups of the B ring of anthocyanidins, which is the chromophore of anthocyanins, increases, blue color becomes more intense, and major anthocyanidins in the form of delphinidin, cyanidin and pelargonidin have increasing numbers of hydroxyl groups in that order.

Anthocyanins derived from delphinidin accumulate in many blue flowers. In addition, as the number of aromatic acyl groups (such as coumaroyl groups or caffeoyl groups) that modify anthocyanins increases, the color of anthocyanins is known to become increasingly blue (namely, maximum absorbance shifts to a longer wavelength), and anthocyanin stability is known to increase. In particular, anthocyanins in which multiple aromatic acyl groups are bonded are referred to as acylated anthocyanins, and are contained in the blue petals of gentians and butterfly peas (see Non-Patent Document 1).

Enzymes involved in anthocyanin biosynthesis and genes that encode those enzymes are previously been isolated from numerous plants (see Non-Patent Document 1). The color of anthocyanins is dependent on such factors as the pH of vacuoles in which they are localized, other flavonoids and metal ions. Namely, anthocyanins appear red if vacuole pH is low, and become blue as vacuole pH becomes neutral. In addition, flavones and flavonols are referred to as co-pigments, and when present with anthocyanins, have the effect of causing the anthocyanins to appear blue. Moreover, iron and aluminum ions are known to cause anthocyanins to become blue by coordinating with hydroxyl groups of the B ring of anthocyanins.

Although plants produce flowers of various colors, there are only a few species capable of producing flowers of all colors. This is because pigments that can be synthesized by a species are determined genetically. For example, there are no blue or violet varieties of roses, carnations, chrysanthemums, lilies and gerbera. The main reason for this is that these plants do not have the flavonoid 3',5'-hydroxylase (F3'5'H) gene required to synthesize delphinidin. Therefore, several attempts to produce blue flowers by inducing the production of delphinidin by expressing F3'5'H gene have been reported.

When F3'5'H gene is made to be expressed in petunias lacking F3'5'H, the amount of anthocyanins derived from delphinidin increases (see Non-Patent Document 2 and Non-Patent Document 3). In addition, when F3'5'H gene is expressed in tobacco (Nicotiana tabacum) that accumulates cyanidin, delphinidin is synthesized and flowers take on a somewhat bluish tint (see Non-Patent Document 3). In the case of having expressed F3'5'H gene derived from campanula, eustoma or petunia in tobacco, F3'5'H derived from campanula produced delphinidin with the greatest efficiency (see Non-Patent Document 4). In addition, in the case of having expressed the F3'5'H gene of butterfly pea or verbena in verbena, the case of having expressed the gene derived from butterfly pea yielded the larger production volume, and flower color underwent a definite change (see Non-Patent Document 5). When F3'5'H gene derived from petunia, gentian, butterfly pea, cineraria, eustoma, pansy and lavender were expressed in roses, delphinidin was produced at a level equal to 10% of the total amount of anthocyanidins only in the case of having expressed the F3'5'H gene derived from pansy, thereby indicating that pansy-derived F3'5'H gene functions favorably in roses (see Patent Document 4).

A genetically modified chrysanthemum that accumulated 50% or more of delphinidin and in which flower color changed to blue was obtained for the chrysanthemum varieties of Improved Reagan and Dark Splendid Reagan by transcribing pansy-derived F3'5'H gene using rose chalcone synthase promoter (see Patent Document 5). When F3'5'H gene derived from such plants as campanula, verbena, cineraria, pansy, petunia or lobelia was expressed using a promoter derived from chrysanthemum-derived flavonone 3-hydroxylase gene and a 5'-untranslated region of tobacco alcohol dehydrogenase gene, the F3'5'H genes derived from campanula, verbena, cineraria and pansy functioned comparatively well, and delphinidin was produced at a level equal to 25% of the total amount of anthocyanidins. In chrysanthemums, the campanula-derived F3'5'H gene functioned most favorably, and delphinidin was produced at a level of 75% of the total amount of anthocyanidins (see Non-Patent Document 16).

On the basis of these reported examples, although examples have been reported of producing delphinidin by expressing a heterologous (foreign) gene in plants, since it is difficult to predict which F3'5'H gene will actually be expressed in the petals of a plant of interest when F3'5'H gene derived from a certain plant is introduced into that plant of interest using a certain promoter, considerable trial-and-error and repeated experiments are required in order to produce delphinidin in petals.

In addition, all of the research results reported in the aforementioned examples were obtained from dicotyledons. There are considerable differences between dicotyledons and monocotyledons even among higher plants.

Firstly, dicotyledons and monocotyledons have various morphological differences. This indicates that monocotyledons have undergone considerable morphological evolution at their origin. Characteristics of monocotyledons consist of: having a single cotyledon, the base of the cotyledon forms a sheath that envelops the other portions of the germ, fibrovascular bundles are sporadically present in cross-sections of the stem, the stem does not become any thicker once it has finished extending (a cambium does not develop), the veins are in the form of parallel lines (parallel veins), a main root does not develop to a great extent, but rather roots are in the form of fibrous roots, the number of tepals, stamen and carpels is a multiple of 3, there is no distinction between petals and sepals (see http://www.fukuoka-edu.ac.jp/-fukuhara /keitai/9-1.html).

Moreover, since monocotyledons and dicotyledons differ considerably in control of gene expression (see Non-Patent Document 6 and Non-Patent Document 7), difficulties are encountered when cultivating genetically modified monocotyledons. However, considerable research and development has been conducted on the monocotyledons of corn and rice, genetically modified corn has already been commercialized in numerous countries.

In this manner, although there are examples of reports describing temporary expression of heterologous (foreign) F3'5'H gene in the petals of monocotyledon flowering plants (see Patent Document 3), there are few reports describing the development of genetically modified monocotyledon flowering plants, and there are still no reports describing the development of monocotyledon flowering plants into which F3'5'H gene has been introduced, namely the development of monocotyledon flowering plants in which flower color has been altered through accumulation of delphinidin in the petals thereof as a result of introducing heterologous F3'5'H gene.

Furthermore, it is described in Non-Patent Document 8 that, when a gene relating to blue pigment synthesis was introduced into red lilies for the purpose of developing blue lilies, lilies were obtained in which the content of blue pigment accounted for one half or more of all pigment. However, the tissue or portion of the lily plants for which blue pigment was analyzed in Non-Patent Document 8 was the petiole. There are many plants in which anthocyanins are not synthesized in flowers even though they are synthesized in leaves (such as thale cress), and there are known cases in which the molecular species of anthocyanins in leaves and anthocyanins in flowers are different. For example, although anthocyanins derived from cyanidin are only contained in the leaves of Sarrecenia purpurea and Sarracenia psitticina, anthocyanins are contained in the flowers thereof that are derived from cyanidin and delphinidin (see Non-Patent Document 17). In addition, control of anthocyanin synthesis most likely also differs according to tissue. For example, although gene c imparts white flowers in morning glories, the stems are colored by anthocyanins while the seeds are black. On the other hand, although gene ca imparts white flowers, the stems are not colored and the seed are white.

Thus, the report describing that delphinidin content accounts for one half or more of all pigment in the petiole of lilies as a result of introducing a heterologous gene does not mean that a method for cultivating blue lilies was established that enables the cultivation of lilies in which delphinidin has accumulated in the petals as a result of introducing that heterologous gene.

In addition, in order to change flower color to blue, the required content of delphinidin is 50% or more, preferably 69% or more, more preferably 70% or more, even more preferably 80% or more, still more preferably 90% or more, more preferably still 95% or more, even more preferably still 99% or more, and most preferably 100%, of the total amount of anthocyanidins. Since it is frequently inadequate to simply overexpress F3'5'H gene in order to achieve this, further contrivances are necessary, including additional gene manipulation.

For example, in the case of carnations, delphinidin content becomes nearly 100% and genetically modified carnations are obtained in which flower color has been changed to blue by expressing both F3'5'H gene and petunia dihydroflavonol 4-reductase (DFR) gene in white carnations deficient in DFR gene (see Patent Document 6 and FIG. 1 of the present application). In addition, in roses, delphinidin content becomes nearly 100% and genetically modified roses are obtained in which flower color has been changed to blue by overexpressing pansy-derived F3'5'H gene and iris-derived DFR gene in addition to suppressing expression of rose DFR gene (see Patent Document 2). In addition, in chrysanthemums, delphinidin content is increased by overexpressing pansy-derived F3'5'H gene and suppressing expression of chrysanthemum endogenous F3'5'H gene (see Patent Document 5).

### PRIOR ART DOCUMENTS

### PATENT DOCUMENTS

Patent Document 1: International Publication No. WO 94/28140 (PCT/AU94/00265, Japanese Unexamined Patent Publication (Translation of PCT Application) No. H8-511683)
Patent Document 2: International Publication No. WO 2005/017147 (PCT/JP2004/001958)
Patent Document 3: Japanese Unexamined Patent Publication No. 2008-253250
Patent Document 4: International Publication No. WO 2004/020637 (PCT/AU03/01111)
Patent Document 5: International Publication No. WO 2009/062253 (PCT/AU2008/001694)
Patent Document 6: International Publication No. WO 96/36716 (PCT/AU96/00296)
Patent Document 7: International Publication No. WO 2007/094521 (PCT/JP2007/053342)
Patent Document 8: International Publication No. WO 2008/156214 (PCT/JP2008/061603)
Patent Document 9: Soon to be published Japanese Patent Application No. 2008-276029

### NON-PATENT DOCUMENTS

Non-Patent Document 1: Plant J. (2008) 54, 737-749
Non-Patent Document 2: Nature (1993) 366, 276-279
Non-Patent Document 3: FEBS Lett. (1999) 461, 241-245
Non-Patent Document 4: Biosci. Biotechnol. Biochem. (2003) 67(1), 161-165
Non-Patent Document 5: Plant Biotechnology (2006) 23, 5-11
Non-Patent Document 6: Protein, Nucleic Acid and Enzyme (1989) 34, 1873-1878
Non-Patent Document 7: Trends in Genetics (1988) 4, 13-18
Non-Patent Document 8: 2006 Annual Report No. I-26 of the Niigata Agricultural Research Institute, (32) Project for the Development of Top Brand Agricultural Products, Cultivation of Blue Lilies
Non-Patent Document 9: Plant Cell Reports (2004) 22, 415-421
Non-Patent Document 10: Plant Cell Reports (2004) 22, 359-364
Non-Patent Document 11: Theor. App. Genet. (1999) 99, 383-390
Non-Patent Document 12: Plant Cell Physiol. (1996) 37, 49-59
Non-Patent Document 13: Clontech, Molecular Breeding (2003) 11, 287-293
Non-Patent Document 14: J. Japan Soc. Hort. Sci. (2008) 77, 94-102
Non-Patent Document 15: Plant J. (2008) 54, 949-962
Non-Patent Document 16: Abstracts of the 2010 Annual Meeting of the Japanese Society of Plant Physiologists, Poster No. P1C012(600), http://www.jspp.org/13member/2010abstract/ pdf/07.pdf Non-Patent Document 17: Hort. Science (2001) 36, 384

### SUMMARY OF THE INVENTION

### PROBLEM TO BE SOLVED BY THE INVENTION

In plants, the anthocyanin-based pigment, delphinidin, is involved in blue color in nearly all cases. Although F3'5'H is essential for the synthesis of delphinidin, this enzyme is absent in lilies. Consequently, blue lilies do not exist. In order to cultivate blue lilies by gene introduction, F3'5'H is required to be highly expressed in lilies. On the other hand, delphinidin content does not increase in lilies synthesizing cyanidin unless synthesis of cyanidin is suppressed.

An object of the present invention is to provide a method for producing lilies that synthesize the blue pigment, delphinidin, at a high content ratio of petal cells by highly expressing F3'5'H derived from a specific species in lily petal cells together with suppressing synthesis of lily endogenous cyanidin.

### MEANS FOR SOLVING THE PROBLEMS

As a result of conducting extensive studies to solve the aforementioned problems, the inventors of the present invention found that, when campanula-derived F3'5'H gene is introduced into the Oriental lily variety "Acapulco", which has pink color and in which cyanidin accounts for nearly all of the anthocyanin-based pigment present, and synthesis of cyanidin is suppressed using RNAi by introducing a fragment of lily F3'H gene, 80% of all anthocyanin-based pigment in the petal cells becomes delphinidin.

The present invention is as indicated below.
[1] A method for producing lilies containing delphinidin in the petals thereof, comprising the following steps:
   introducing into lilies a campanula-derived flavonoid 3',5'-hydroxylase (F3'5'H) gene composed of a nucleotide sequence represented by SEQ ID NO: 1 or SEQ ID NO: 11, or a nucleotide sequence having at least 90% sequence identity with the nucleotide sequence represented by SEQ ID NO: 1 or SEQ ID NO: 11 that also encodes a polypeptide having F3'5'H activity,
   introducing a fragment of lily-derived flavonoid 3'-hydroxylase (F3'H) gene composed of a nucleotide sequence represented by SEQ ID NO: 3 or SEQ ID NO: 16, or a nucleotide sequence having at least 90% sequence identity with the nucleotide sequence represented by SEQ ID NO: 3 or SEQ ID NO: 16 that also encodes a polypeptide having F3'H activity, and
   introducing a fragment of lily-derived dihydroflavonol 4-reductase (DFR) gene composed of a nucleotide sequence represented by SEQ ID NO: 13 or a nucleotide sequence having at least 90% sequence identity with the nucleotide sequence represented by SEQ ID NO: 13 that also encodes a polypeptide having DFR activity, to suppress expression of endogenous DFR that acts on cyanidin synthesis in lily petals, and simultaneously introducing an iris-derived foreign DFR gene; and
   synthesizing delphinidin by the action of the introduced F3'5'H gene while suppressing expression of endogenous F3'H gene that acts on cyanidin synthesis in lily petals.
[2] The method described in [1] above, wherein the suppression of endogenous F3'H expression is carried out in the RNAi method.
[3] The method described in [1] or [2] above, wherein the campanula-derived F3'5'H gene is composed of a nucleotide sequence having at least 95% sequence identity with the nucleotide sequence represented by SEQ ID NO: 1 or SEQ ID NO: 11 that also encodes a polypeptide having F3'5'H activity.
[4] The method described in [1] or [2] above, wherein the campanula-derived F3'5'H gene is composed of a nucleotide sequence represented by SEQ ID NO: 1 or SEQ ID NO: 11.
[5] The method described in any of [1] to [4] above, wherein the fragment of lily-derived F3'H gene is composed of a nucleotide sequence having at least 95% sequence identity with the nucleotide sequence represented by SEQ ID NO: 3 or SEQ ID NO: 16 that also encodes a polypeptide having F3'H activity.
[6] The method described in any of [1] to [4] above, wherein the fragment of lily-derived F3'H gene is composed of a nucleotide sequence represented by SEQ ID NO: 3 or SEQ ID NO: 16.
[7] The method described in any of [1] to [6] above, further comprising: using a lily DFR promoter sequence represented by SEQ ID NO: 24.
[8] A lily, tissue thereof, portion thereof, vegetatively propagated plant thereof or progeny thereof, which contains delphinidin in the petals thereof produced according to the method described in any of [1] to [7] above.
[9] The lily, tissue thereof, portion thereof, vegetatively propagated plant thereof or progeny thereof described in [8] above, wherein the tissue or portion thereof is a cut flower.

### EFFECTS OF THE INVENTION

According to the method for producing lilies containing delphinidin in the petals thereof according to the present invention, a blue pigment present in lily petal cells in the form of delphinidin can be made to accumulate at a high content ratio, thereby making it possible to produce previously non-existent lilies that produce blue flowers.

### BRIEF DESCRIPTION OF THE DRAWINGS

FIG. 1 indicates the biosynthesis pathway of anthocyanins.
FIG. 2 indicates a map of a binary vector Tr3.
FIG. 3 indicates maps of binary vectors Tr7 to Tr11.

### EMBODIMENTS OF THE INVENTION

The following provides a detailed explanation of the present invention.

The present invention relates to a method for producing lilies containing delphinidin in the petals thereof, comprising the following steps:
introducing into lilies a campanula-derived flavonoid 3',5'-hydroxylase (F3'5'H) gene composed of a nucleotide sequence represented by SEQ ID NO: 1 or SEQ ID NO: 11, or a nucleotide sequence having at least 90% sequence identity with the nucleotide sequence represented by SEQ ID NO: 1 or SEQ ID NO: 11 that also encodes a polypeptide having F3'5'H activity,
introducing a fragment of lily-derived flavonoid 3'-hydroxylase (F3'H) gene composed of a nucleotide sequence represented by SEQ ID NO: 3 or SEQ ID NO: 16, or a nucleotide sequence having at least 90% sequence identity with the nucleotide sequence represented by SEQ ID NO: 3 or SEQ ID NO: 16 that also encodes a polypeptide having F3'H activity, and
introducing a fragment of lily-derived dihydroflavonol 4-reductase (DFR) gene composed of a nucleotide sequence represented by SEQ ID NO: 13 or a nucleotide sequence having at least 90% sequence identity with the nucleotide sequence represented by SEQ ID NO: 13 that also encodes a polypeptide having DFR activity, to suppress expression of endogenous DFR that acts on cyanidin synthesis in lily petals, and simultaneously introducing an iris-derived foreign DFR gene; and
synthesizing delphinidin by the action of the introduced F3'5'H gene while suppressing expression of endogenous F3'H gene that acts on cyanidin synthesis in lily petals.

Examples of the aforementioned nucleotide sequence having sequence identity of at least 90% with a nucleotide sequence represented by SEQ ID NO: 1 or SEQ ID NO: 11 that also encodes a polypeptide having flavonoid 3'5'-hydroxylase (F3'5'H) activity include DNA having sequence identity of least 91%, 92%, 93%, 94%, 95%, 96%, 97%, 98% and 99% or more with DNA composed of a nucleotide sequence represented by SEQ ID NO: 1 when calculated using analysis software such as BLAST or FASTA.

Similarly, examples of the aforementioned nucleotide sequence having at least 90% sequence identity with a nucleotide sequence represented by SEQ ID NO: 3 or SEQ ID NO: 16 that also encodes a polypeptide having flavonoid 3'-hydroxylase (F3'H) activity include DNA having sequence identity of at least 91%, 92%, 93%, 94%, 95%, 96%, 97%, 97%, 98% and 99% or more with DNA composed of a nucleotide sequence represented by SEQ ID NO: 3 when calculated using analysis software such as BLAST or FASTA.

The nucleotide sequence having at least 90% sequence identity with a nucleotide sequence represented by SEQ ID NO: 1 or SEQ ID NO: 11 that has flavonoid 3',5'-hydroxylase (F3'5'H) activity can also be a nucleotide sequence in which one or more nucleotides have been deleted, substituted or added in a nucleotide sequence represented by SEQ ID NO: 1 or SEQ ID NO: 11 that also encodes a polypeptide having flavonoid 3',5'-hydroxylase (F3'5'H) activity. Here, "one or more" in DNA or a nucleotide sequence refers to 1 to 20, preferably 1 to 10, more preferably 1 to 5, even more preferably 1 to 4, and still more preferably 1 to 3 of the bases in the nucleotide sequence represented by SEQ ID NO: 1 being deleted, substituted or added provided it is within the range of having the aforementioned enzyme activity. In addition, the terms "deletion", "substitution" and "addition" refer to the formation of a nucleotide sequence that encodes a protein having properties similar to a protein (or polypeptide) (SEQ ID NO: 2) encoded by the nucleotide sequence represented by SEQ ID NO: 1.

Similarly, the nucleotide sequence having at least 90% sequence identity with a nucleotide sequence represented by SEQ ID NO: 3 or SEQ ID NO: 16 that has flavonoid 3'-hydroxylase (F3'H) activity can also be a nucleotide sequence in which one or more nucleotides have been deleted, substituted or added in a nucleotide sequence represented by SEQ ID NO: 3 or SEQ ID NO: 16 that also encodes a polypeptide having flavonoid 3'-hydroxylase (F3'H) activity. Here, "one or more" in DNA or a nucleotide sequence refers to 1 to 20, preferably 1 to 10, more preferably 1 to 5, even more preferably 1 to 4, and still more preferably 1 to 3 of the nucleotides in the nucleotides sequence represented by SEQ ID NO: 3 or SEQ ID NO: 16 being deleted, substituted or added provided it is within the range of having the aforementioned enzyme activity. In addition, the terms "deletion", "substitution" and "addition" refer to the formation of a nucleotide sequence that encodes a protein having properties similar to a protein (or polypeptide) (SEQ ID NO: 4 or SEQ ID NO: 17) encoded by the nucleotide sequence represented by SEQ ID NO: 1 or SEQ ID NO: 16.

The nucleotide sequence having sequence identity of at least 90% with a nucleotide sequence represented by SEQ ID NO: 1 or SEQ ID NO: 11 that also encodes a polypeptide having flavonoid 3',5'-hydroxylase (F3'5'H) activity can be a nucleotide sequence capable of hybridizing under stringent conditions with a complementary strand of a nucleotide sequence represented by SEQ ID NO: 1 or SEQ ID NO: 11 that also encodes a polypeptide having flavonoid 3'5'-hydroxylase (F3'5'H) activity.

Similarly, the nucleotide sequence having sequence identity of at least 90% with a nucleotide sequence represented by SEQ ID NO: 3 or SEQ ID NO: 16 that also encodes a polypeptide having flavonoid 3'-hydroxylase (F3'H) activity can be a nucleotide sequence capable of hybridizing under stringent conditions with a complementary strand of a nucleotide sequence represented by SEQ ID NO: 3 that also encodes a polypeptide having flavonoid 3'-hydroxylase (F3'H) activity.

Here, although the temperature and salt concentration during hybridization, and preferably during washing as well, can be suitably set corresponding to the DNA that is hybridized to a complementary strand of DNA composed of SEQ ID NO: 1 or 11 or SEQ ID NO: 3 or 16, "stringent conditions" with respect to a nucleotide or DNA (sequence) refers to conditions described in, for example, "Molecular Cloning: A Laboratory Manual (2nd Edition)" edited by Sambrook et al. (Cold Spring Harbor Laboratory Press (1989). More specifically, this refers to a step consisting of (i) incubating overnight at 42°C with a probe in a solution containing 6xSSC (composition of 1xSSC: 0.15 M NaCl, 0.015 M sodium citrate, pH 7.0), 0.5% SDS, 5x Denhardt's solution, 100 µg/mL denatured fragmented salmon sperm DNA and 50% formaldehyde, and (ii) removing non-specifically hybridized probe by washing, wherein from the viewpoint of further enhancing accuracy, washing under conditions of lower ionic strength such as 2xSSC and more stringently at 0.1xSSC, and/or washing at a high temperature, such as 40°C lower than the Tm value of the nucleic acid used, more stringently at 30°C lower, even more stringently at 25°C lower and still more stringently at 10°C lower, and more specifically, although varying according to the Tm value of nucleic acid used, washing under conditions of 25°C or higher, more stringently at 37°C or higher, even more stringently at 42°C or higher, still more stringently at 50°C or higher, and even more stringently still at 60°C or higher. Tm is determined from, for example, the formula Tm = 81.5 + 16.6(log[Na⁺]) + 0.41(%G+C) - (600/N) (wherein, N represents oligonucleotide length, and %G+C represents the contents of guanine and cytosine residues in the oligonucleotide). Hybridization conditions may be set by, for example, referring to the aforementioned publication. Furthermore, the procedure described in the present description can be suitably carried out by referring to the aforementioned publication.

Suppression of expression of the endogenous flavonoid 3'-hydroxylase (F3'H) acting on cyanidin synthesis in lily petals by introducing a fragment of lily-derived F3'H gene composed of a sequence represented by SEQ ID NO: 3 or SEQ ID NO: 16 or a sequence having at least 90% sequence identity with a nucleotide sequence represented by SEQ ID NO: 3 or SEQ ID NO: 16 that also encodes a polypeptide having F3'H activity may be carried out by antisense suppression, sense suppression (co-suppression) or RNAi and the like, the RNAi method is preferable.

The present invention also comprises inserting a fragment of lily-derived dihydroflavonol 4-reductase (DFR) gene composed of a nucleotide sequence represented by SEQ ID NO: 13 or a nucleotide sequence having at least 90% sequence identity with the nucleotide sequence represented by SEQ ID NO: 13 that also encodes a polypeptide having DFR activity, suppressing expression of endogenous DFR that acts on cyanidin synthesis in lily petals, and simultaneously introducing an iris-derived foreign DFR gene. The suppression of expression of endogenous DFR is preferably carried out in the RNAi method.

The present invention also relates to the aforementioned method further comprising the step of using a lily DFR promoter represented by SEQ ID NO: 24.

Furthermore, the definitions of the deletion, substitution or addition of one or more nucleotides in a nucleotide sequence with respect to SEQ ID NO: 1, 11, 3 and 16, sequence identify and hybridization are similarly also applied to SEQ ID NO: 13 and 24, and instead of the nucleotide sequences represented by SEQ ID NO: 13 and 24, for example, a nucleotide sequence having sequence identity of at least 90%, 95% or 99% therewith is also within the scope of the present invention.

A lily containing delphinidin in the petals thereof produced according to the method for producing lilies containing delphinidin in the petals thereof according to the present invention, tissue thereof, a portion thereof, a vegetatively propagated plant thereof or progeny thereof, is within the scope of the present invention. The tissue or portion thereof can preferably be a cut flower.

### EXAMPLES

Although the following provides a more detailed explanation of the present invention through examples thereof, the scope of the present invention is not intended to be limited by these examples.

### [Reference Example 1: Expression of Periwinkle F3'5'H Gene in Lilies]

Plasmid pNAVFH (see Non-Patent Document 9, binary vector for expressing periwinkle F3'5'H gene in plants) was introduced into Agrobacterium strain EHA101. The lily variety, Acapulco (pink flowers), was transformed using this genetically modified Agrobacterium. Although lily transformation was carried out with the method described in Non-Patent Document 10, for example, the method used is not limited thereto.

Two to three years are required after acquiring a plant tissue culture of the transformed lilies in order to investigate whether the introduced F3'5'H gene functions in lilies and induces accumulation of delphinidin. Analyzing anthocyanins present in cultured petioles by focusing on observation of anthocyanin coloring in the petioles at least makes it possible to rapidly determine whether the introduced gene has not functioned or has hardly functioned at all. Plant tissue cultures of the transformed lilies of the resulting 19 cutivars were acquired. Approximately 0.5 g of these petioles in which anthocyanin coloring was observed were recovered and subjected to anthocyanidin analysis using the method described in Patent Document 2. As a result, there were no cultivars observed in which delphinidin was detected.

### [Reference Example 2: Expression of Cineraria F3'5'H Gene in Lilies]

An approximately 1.7 kb DNA fragment obtained by digesting plasmid pSPB2774 containing cineraria-derived F3'5'H cDNA Ci5a18 (see Patent Document 9) with SmaI and XhoI, a DNA fragment containing cauliflower mosaic virus 35S promoter (to also be referred to as "35S promoter") obtained by digesting plasmid pBI1221 with HindIII and BamHI, and a DNA fragment containing the replicon of a DNA fragment obtained by digesting plasmid pSPB176 (see Patent Document 7) with HindIII and SalI, were ligated to obtain plasmid pSPB3472. A DNA fragment obtained by digesting this plasmid with AscI and PacI (containing a sequence in which 35S promoter, Ci5a18 and nopaline synthase terminator are ligated) was ligated with a DNA fragment obtained by digesting #493 with AscI and PacI to obtain plasmid pSPB3376. This was then introduced into lilies using Agrobacterium as described in Reference Example 1 to acquire plant tissue cultures of 65 cultivars of transformed lilies. Although leafstalks in which anthocyanins had accumulated were acquired from 23 of these cultivars, there were no cultivars obtained in which delphinidin had accumulated.

### [Reference Example 3: Expression of Gentian F3'5'H Gene in Lilies]

An approximately 1.7 kb DNA fragment obtained by digesting plasmid pGHF48 containing gentian F3'5'H cDNA (see Patent Document 4) was ligated with plasmid pSPB176 digested with BamHI and SalI to obtain plasmid pSPB3329. The promoter portion was removed by digesting this with HindIII and BamHI. Plasmid pSPB3473 was then obtained by introducing a DNA fragment obtained by digesting pBI1221 with HindIII and BamHI (containing 35S promoter) therein. A DNA fragment obtained by digesting this plasmid with AscI and PacI (containing a sequence in which 35S promoter, gentian F3'5'H cDNA and nopaline synthase terminator are ligated) was ligated with a DNA fragment obtained by digesting pSPB493 with AscI and PacI to obtain plasmid pSPB3378. This was then introduced into lilies using Agrobacterium as described in Reference Example 1 to acquire plant tissue cultures of 65 strains of transformed lilies. Petioles in which anthocyanins had accumulated were acquired from 26 of these cultivars. The delphinidin accumulation ratio (ratio of the amount of delphinidin accumulated to the total amount of accumulated anthocyanidins) only reached a maximum of 1%.

### [Reference Example 4: Expression of Lavender F3'5'H Gene in Lilies]

An approximately 1.7 kb DNA fragment obtained by digesting plasmid pLBG8 containing lavender F3'5'H cDNA (see Patent Document 4) with BamHI and XhoI was ligated with plasmid pSPB176 digested with BamHI and SalI to obtain plasmid pSPB2772. A DNA fragment containing a sequence in which lavender F3'5'H cDNA and nopaline synthase terminator were ligated and obtained by digesting this plasmid with BamHI and PacI was recovered. This DNA fragment was ligated with a fragment containing the replicon of a DNA fragment obtained by digesting plasmid pSPB3472 (3377) with BamHI and PacI to obtain plasmid pSPB3471. A DNA fragment obtained by digesting this plasmid with AscI and PacI (containing a sequence in which 35S promoter, gentian F3'5'H cDNA and nopaline synthase terminator are ligated) was ligated with a DNA fragment obtained by digesting pSPB493 with AscI and PacI to obtain plasmid pSPB3376. This was then introduced into lilies using Agrobacterium as described in Reference Example 1 to acquire plant tissue cultures of 23 sultivars of transformed lilies. Petioles in which anthocyanins had accumulated were acquired from 20 of these cultivars. The delphinidin accumulation ratio (ratio of the amount of delphinidin accumulated to the total amount of accumulated anthocyanidins) only reached a maximum of 1%.

### [Reference Example 5: Expression of Butterfly Pea F3'5'H Gene in Lilies]

Introduction of butterfly pea F3'5'H gene (DNA Database Accession No. AB234897) into verbena has been reported to function better and result in a higher delphinidin content and a more distinct change in flower color than in the case of introducing the F3'5'H gene of verbena per se (see Non-Patent Document 5). Plasmid pSPB748 (see Non-Patent Document 5) was digested with EcoRI, subjected to blunting and digested with BamHI followed by recovering a DNA fragment containing butterfly pea-derived F3'5'H cDNA. On the other hand, a DNA fragment was obtained by digesting pSPB176 with SalI, blunting and digesting with BamHI. These two DNA fragments were ligated to obtain plasmid pSPB3166. A DNA fragment containing F3'5'H cDNA obtained by digesting pSPB3166 with AscI and PacI was inserted into a DNA fragment obtained by digesting binary vector pSBP3166, which contains a gene imparting hygromycin resistance (see Non-Patent Document 11), with AscI and PacI. The resulting binary vector was designated as pSBP3169. In plants transformed with this binary vector, butterfly pea F3'5'H gene is transcribed under the control of cauliflower mosaic virus 35S promoter to which an enhancer has been added (see Non-Patent Document 12).

This was then introduced into lilies using Agrobacterium as described in Reference Example 1, and plant tissue cultures of 23 cultivars of transformed lilies were acquired in anticipation of production of delphinidin following introduction of butterfly pea F3'5'H gene into lilies. Although petioles in which anthocyanins had accumulated were acquired from 6 of these cultivars, there were no cultivars obtained in which delphinidin had accumulated.

### [Reference Example 6: Expression of Pansy F3'5'H Gene in Lilies]

In the case of having introduced F3'5'H genes derived from several plants into roses, only pansy-derived F3'5'H gene (DNA Database Accession No. AB332097) resulted in favorable accumulation of delphinidin and caused a distinct change in flower color (see Patent Document 4). In addition, this gene also causes delphinidin to accumulate in chrysanthemum petals and causes flower color to change to blue (see Patent Document 5). On the basis of these facts, pansy-derived F3'5'H gene was presumed to be suitable for producing delphinidin in different plants.

A DNA fragment obtained by digesting plasmid pSPB575 containing pansy F3'5'H cDNA (see Patent Document 8) with AscI and PacI and a DNA fragment obtained by digesting plasmid pSPB3166 with AscI and PacI were ligated to obtain binary vector pSBP3138. In plants transformed with this binary vector, pansy F3'5'H gene is transcribed under the control of cauliflower mosaic virus 35S promoter to which an enhancer has been added.

This was then introduced into lilies using Agrobacterium as described in Reference Example 1, and plant tissue cultures of 25 cultivars of transformed lilies were acquired in anticipation of production of delphinidin following introduction of pansy F3'5'H gene into lilies. Although petioles in which anthocyanins had accumulated were acquired from 12 of these cultivars, there were no cultivars obtained in which delphinidin had accumulated.

### [Example 1]

### (1) Isolation of F3'5'H Gene from Campanula Genomic DNA

Chromosomal DNA was extracted from leaves of Campanula (Campanula medium) using DNeasy (Qiagen Corp.). Nucleotides CFH1L: 5'-CATGTCTATAGACATATCCACCCTCTTCTATGAAC-3' (SEQ ID NO: 5, underlines indicate start codon) and cCFH1L: 5'-GCCTAGACAGTGTAAGAACTTGGAGGCAATCTTGG-3' (SEQ ID NO: 6) were synthesized based on the base sequence of campanula (Campanula medium) F3'5'H cDNA (DNA Database Accession No. D14590). PCR was carried out using 100 ng of chromosomal DNA as template and using the aforementioned nucleotides as primers. Takara LA PCR (Takara Bio Inc.) was used to carry out PCR under the conditions recommended by the manufacturer. The resulting base sequence is represented by SEQ ID NO: 1. Amino acid SEQ ID NO: 2 encoded by this base sequence is the same as that registered for DNA Database Accession No. D14590.

### (2) Construction of Binary Vector Containing F3'5'H Gene from Campanula Genomic DNA

Plasmid pBI221 (see Non-Patent Document 13) was digested with restriction enzymes SmaI and SacI followed by blunting using a blunting kit (Takara Bio Inc.) and subjecting to ligation. The resulting plasmid was digested with restriction enzyme BamHI and blunted. This DNA fragment was then reacted with DNA polymerase in the presence of deoxythymidine triphosphate to add a single deoxythymidine base to the 3'-end of this DNA fragment. This DNA fragment was then ligated with PCR fragments derived from the aforementioned campanula chromosomal DNA. Among the resulting plasmids, the plasmid in which the start codon of campanula F3'5'H gene was inserted in a direction close to a 35S promoter sequence was designated as p35S-CFH-1. This plasmid was digested with PvuII and separated by agarose gel electrophoresis to recover a DNA fragment containing campanula F3'5'H gene.

Plasmid pNAVFH (see Non-Patent Document 9, binary vector for expressing periwinkle F3'5'H gene in plants) was digested with restriction enzymes HindIII and XbaI, blunted and dephosphorylated. This was then separated by agarose gel electrophoresis followed by recovery of the DNA fragment having the largest molecular weight. This DNA fragment was ligated with the aforementioned DNA fragment containing campanula F3'5'H gene, and the resulting binary vector was designated as pNARI-CFH.

### (3) Isolation of Lily F3'H Gene Fragment (Construction of pCR2.1-AP24-1)

Oligonucleotides F3H2: 5'-YTSGCYGGWGTWTTYAACRTHGG-3', equivalent to LAGVFNIG in the sequence of thale cress (SEQ ID NO: 7), and cF3H4: 5'-GGRTCNCGRGCWATGGCCCA-3', equivalent to NIWAIARDP in the sequence of thale cress (SEQ ID NO: 8), were synthesized based on the previously reported F3'H amino acid sequences of thale cress (DNA Database Accession No. AF271651), aster (AF313488), soybean (AF499731), perilla (AB045593), rice (AC021892) and torenia (AB057672). Here, Y represents a mixture of C and T, W represents a mixture of A and T, S represents a mixture of C and G, R represents a mixture of A and G, H represents a mixture of A, T and C, and N represents a mixture of G, A, T and C. RNA was extracted from the petals of the lily variety, Acapulco (Yamaki Noen Co., Ltd.), using RNeasy (Qiagen Corp.). RT-PCR was carried out using this RNA as template and using F3H2 and oligo(dT) as primers. RT-PCR was carried out using an RT-PCR kit (Invitrogen Corp.). PCR was carried out as previously described using the amplified DNA fragment as template and using F3H2 and cF3H4 as primers. The resulting DNA fragment was designated as SEQ ID NO: 3. A DNA fragment having this sequence was then ligated to plasmid pCR2.1 (Invitrogen Corp.). The resulting plasmid was designated as pCR2.1-AP24-1.

### (4) Construction of Plasmid pNARI-Tr3

Plasmid pCR2.1-AP24-1 was cleaved with SpeI and XbaI, blunted and separated by agarose gel electrophoresis. A DNA fragment having a length of 665 bp was recovered from the resulting DNA fragments. This DNA fragment was ligated with a DNA fragment obtained by cleaving plasmid pBI221 with restriction enzymes ScaI and MluI, blunting and subjecting to dephosphorylation treatment. The resulting plasmid was designated as p35S-GUSL-AP24.

Plasmid pCR2.1-AP24-1 was cleaved with SpeI and NotI, blunted and separated by agarose gel electrophoresis. A 647 bp DNA fragment was recovered from the resulting DNA fragments. This DNA fragment was ligated with a DNA fragment obtained by digesting plasmid p35S-GUSL-AP24 with SmaI and subjecting to dephosphorylation treatment, and the resulting plasmid was designated as plasmid p35S-F3'HTr2.

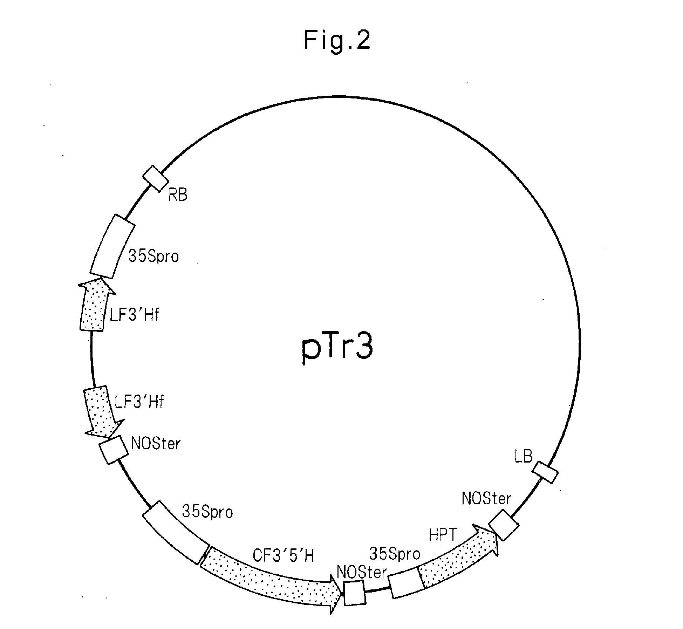

Plasmid p35S-F3'HTr2 was cleaved with PvuII and separated by agarose gel electrophoresis followed by recovery of an approximately 3.3 kb DNA fragment. On the other hand, plasmid pNARI-CFH was cleaved with ClaI and NheI, blunted, dephosphorylated and separated by agarose gel electrophoresis followed by recovery of a DNA fragment containing the replicon. The recovered DNA fragment was ligated with the aforementioned approximately 3.3 kb DNA fragment. The resulting plasmid or binary vector was designated as pNARI-Tr3 (which may also be simply referred to as "Tr3"). As shown in FIG. 2, this binary vector has a hygromycin phosphotransferase gene on T-DNA as a selection marker, and it was intended to suppress expression of lily F3'H gene by constitutively expressing campanula F3'5'H gene and transcribing double-stranded RNA of lily F3'H gene.

### (5) Lily Transformation

Binary vectors pNARI-CFH and pNARI-Tr3 were each introduced into Agrobacterium strain EHA101. Lily variety Acapulco (pink flowers) was transformed using these genetically modified Agrobacterium as described in Reference Example 1. In the case of using Agrobacterium containing pNARI-CFH, plant tissue cultures of 31 strains of lilies were acquired. Analysis of anthocyanidins in the leafstalks where anthocyanins had accumulated revealed that delphinidin had accumulated at a level of 5% or more of the total amount of anthocyanidins in 4 strains. The maximum delphinidin content was 18%.

On the other hand, in the case of using Agrobacterium containing pNART-Tr3, plant tissue cultures of 40 strains of lilies were acquired. Analysis of anthocyanidins in the leafstalks where anthocyanins had accumulated revealed that delphinidin had accumulated at a level of 5% or more of the total amount of anthocyanidins in 13 cultivars as shown in the following Table 1. When several of these genetically modified lilies were allowed to bloom in a greenhouse enabling the cultivation of genetically modified plants, the content of delphinidin in the petals reached a maximum of 80%, and in contrast to the original color of this variety having a color chart number of N74D as classified by the British Royal Horticultural Society, the color of the genetically modified lilies become bluer in comparison with the original color, and demonstrated a color chart number of 81C.

**[Table 1]**

| Individual No. | Petiole Anthocyanidin Analysis (delphinidin/cyanidin/pelargonidin: %) | Petal Anthocyanidin Analysis (delphinidin/cyanidin/pelargonidin: %) |
|---|---|---|
| AP Tr3 No. 1 | 4/96/0 | 0/96/4 |
| AP Tr3 No. 2 | 3/97/0 | 1/84/15 |
| AP Tr3 No. 3 | 26/63/11 | Did not bloom |
| AP Tr3 No. 4 | 0/100/0 | Did not bloom |
| AP Tr3 No. 5 | 53/39/8 | 80/13/7 (090323) |
| AP Tr3 No. 6 | 7/91/2 | Did not bloom |
| AP Tr3 No. 7 | 0/100/0 | Did not bloom |
| AP Tr3 No. 8 | 0/100/0 | Did not bloom |
| AP Tr3 No. 9 | 0/100/0 | Did not bloom |
| AP Tr3 No. 10 | 0/100/0 | 0/99/1 (091020) |
| AP Tr3 No. 11 | 0/100/0 | 7/78/15 (091020) |
| AP Tr3 No. 12 | 68/25/7 | 65/20/15 (080516) |
| AP Tr3 No. 13 | 0/100/0 | Did not bloom |
| AP Tr3 No. 14 | 50/40/10 | 12/53/32 (080516) |
| AP Tr3 No. 15 | 30/68/2 | Did not bloom |
| AP Tr3 No. 16 | 3/97/0 | 3/94/3 (091020) |
| AP Tr3 No. 17 | 20/77/3 | Did not bloom |
| AP Tr3 No. 18 | 50/43/7 | 21/79/0 |
| AP Tr3 No. 19 | 0/100/0 | Did not bloom |
| AP Tr3 No. 20 | 0/100/0 | Did not bloom |
| AP Tr3 No. 21 | 45/53/2 | Dead |
| AP Tr3 No. 22 | 25/70/5 | 0/82/18 |
| AP Tr3 No. 23 | 1/99/0 | 0/84/16 |
| AP Tr3 No. 24 | 5/93/2 | Did not bloom |
| AP Tr3 No. 25 | 9/89/2 | Did not bloom |
| AP Tr3 No. 26 | 30/67/3 | Dead |
| AP Tr3 No. 27 | 4/95/1 | 1/85/14 |
| AP Tr3 No. 28 | 2/98/0 | Did not bloom |
| AP Tr3 No. 29 | 3/97/0 | Did not bloom |
| AP Tr3 No. 30 | 30/69/1 | Did not bloom |
| AP Tr3 No. 31 | 4/94/2 | Did not bloom |
| AP Tr3 No. 32 | 2/98/0 | Did not bloom |
| AP Tr3 No. 33 | 10/90/0 | 53/39/8 |
| AP Tr3 No. 34 | 2/98/0 | Did not bloom |
| AP Tr3 No. 35 | 0/100/0 | Did not bloom |
| AP Tr3 No. 36 | 0/100/0 | Did not bloom |
| AP Tr3 No. 37 | 16/84/0 | Did not bloom |
| AP Tr3 No. 38 | 0/100/0 | Did not bloom |
| AP Tr3 No. 39 | 0/100/0 | Did not bloom |
| AP Tr3 No. 40 | 0/100/0 | Did not bloom |

In addition, pelargonidin was also detected in the genetically modified plants that is not observed in the host. This is presumed to be the effect of suppression of F3'H gene.

Similarly, Tiara (Yamaki Noen Co., Ltd.) (to also be referred to as "TI"), lily variety R094-19 (Niigata Agricultural Research Institute) (to also be referred to as "RO") and MP8 (provided by Mr. Miwa Fujii) were also transformed using Agrobacterium containing pNARI-Tr3, and 38, 82 and 12 genetically modified cultivars, respectively, were obtained. Analysis of anthocyanidins in the petioles where anthocyanins had accumulated in these cultivars revealed that the average content of delphinidin was 24%, 8% and 11%, respectively, and the maximum value of delphinidin content was 68%, 53% and 35%, respectively.

Analysis values and flower color for those TI (Tiara) and R0 (R094-19) that bloomed are shown in the following Table 2. The maximum content of delphinidin in petals of the genetically modified tiara that bloomed was 74% (individual no. TI Tr3 No. 42). The maximum content of delphinidin in petals of genetically modified R094-19 that bloomed was 15% (individual no. RO Tr3 No. 9).

**[Table 2]**

| Individual No. | Delphinidin Content (%) | Color Chart No. |
|---|---|---|
| TI Tr3 No. 4 | 57 | 75D |
| TI Tr3 No. 24 | 47 | 76C |
| TI Tr3 No. 42 | 74 | 75D |
| TI Tr3 No. 43 | 0 | 84C |
| TI Tr3 No. 59 | 23 | 76D |
| TI Non-transformed | 0 | 75C |
| RO Tr3 No. 1 | 0 | 75B |
| RO Tr3 No. 9 | 15 | 75C |
| RO Tr3 No. 12 | 0 | NN155C |
| RO Tr3 No. 13 | 0 | NN155C |
| RO Tr3 No. 18 | 0 | NN155C |
| RO Non-transformed | 0 | 75B |

Moreover, anthocyanins present in host Acapulco variety and a genetically modified lily (also referred to as "Acapulco Tr3-5") were analyzed by LC-FTICR-MS (see Non-Patent Document 14 and Non-Patent Document 15). The use of this technique makes it possible to precisely measure the mass spectra of anthocyanins and obtain MS/MS spectra by tandem mass spectrometry. In the host Acapulco variety, peaks corresponding to cyanidin, having detected m/z of 595.166612 (MS/MS m/z: 287.1 (10%), 432.8 (0.9%), 449.1 (14.7%) (figures in parentheses indicate relative values of peak intensity), bound with 1 equivalent of rhamnose and glucose comprised the major anthocyanins. On the other hand, in the case of Acapulco Tr3-5, two peaks corresponding to delphinidin, having detected m/z of 611.160529 (MS/MS mz: 303.1 (100%), 449.2 (1.1%), 465.1 (10.8%)) and 611.160902 (MS/MS m/z: 303.1 (100%), 449.1 (0.7%), 465.1 (12.3%)), bound with 1 equivalent of rhamnose and glucose were observed that are not observed in the host Acapulco variety. Moreover, an increase was observed in a peak corresponding to pelargonidin, which is only observed at extremely low levels in the host Acapulco variety, bound with one equivalent of rhamnose and glucose (detected m/z: 579.171847 (MS/MS m/z: 271.1 (100%), 417.1 (0.8%), 433.1 (18.4%)).

### [Example 2: Expression of Campanula F3'5'H cDNA in Lilies]

### (1) Isolation of Campanula F3'5'H cDNA

Two types of primers consisting of CamF1: 5'-GTGAAGCCACCATGTCTATAG-3' (SEQ ID NO: 9) and CamR1: 5'-GCATTTGCCTAGACAGTGTAAG-3' (SEQ ID NO: 10) were synthesized based on the translation sequence of F3'5'H cDNA of campanula (Campanula medium) registered in the GenBank DNA database (DNA Database Accession No. D14590). RNA was extracted from the petals of commercially available campanula buds using the RNeasy Mini Plant Kit (Qiagen Corp.), and 1st strand DNA was synthesized using an RT-PCR kit. PCR was then carried out using the primers while using this 1st strand DNA as template. The resulting DNA fragments were cloned in pCR-TOPO II. Among these clones, the base sequence of cDNA of clone #4 (designated as pSPB2561) was determined to be SEQ ID NO: 11. In contrast to the F3'5'H registered for DNA Database Accession No. D14590 being composed of 523 residues (SEQ ID NO: 2), the F3'5'H encoded by the cDNA of clone #4 was composed of 521 residues (SEQ ID NO: 12), and demonstrated identity of 95% with the F3'5'H registered for D14590.

Campanula genomic F3'5'H gene is contained in the binary vector introduced into lilies in Example 1. This sequence (SEQ ID NO: 1) has an intron. Binary vectors in which this genomic F3'5'H gene was replaced with a sequence derived from the aforementioned clone #4 (not containing an intron) were constructed. The binary vector not containing F3'5'H double-stranded DNA was designated as pNARI-cCFH (to also be referred to as "cC"), while the binary vector containing F3'H double-stranded DNA was designated as pNARI-cTr3 (to also be referred to as "cTr3").

As shown in the following Table 3, in the case of using Agrobacterium containing pNARI-cCFH, plant tissue cultures of 42 cultivars of genetically modified lilies were acquired. Petioles in which anthocyanins had accumulated were acquired. Analysis of anthocyanidins in the petioles where anthocyanins had accumulated revealed that delphinidin had accumulated at a level of 5% or more of the total amount of anthocyanidins in 16 cultivars. The maximum delphinidin content was 36%.

**[Table 3]**

| Individual No. | Petiole Anthocyanidin Analysis (delphinidin/cyanidin/ pelargonidin: %) |
|---|---|
| AP cC No. 1 | 1/99/0 |
| AP cC No. 2 | 13/87/0 |
| AP cC No. 3 | 1/99/0 |
| AP cC No. 4 | 1/99/0 |
| AP cC No. 5 | 7/93/0 |
| AP cC No. 6 | 1/99/0 |
| AP cC No. 7 | 4/96/0 |
| AP cC No. 8 | 1/99/0 |
| AP cC No. 9 | 5/95/0 |
| AP cC No. 10 | 1/99/0 |
| AP cC No. 11 | 0/100/0 |
| AP cC No. 12 | 0/100/0 |
| AP cC No. 13 | 1/99/0 |
| AP cC No. 14 | 1/99/0 |
| AP cC No. 15 | 1/99/0 |
| AP cC No. 16 | 1/99/0 |
| AP cC No. 17 | 1/99/0 |
| AP cC No. 18 | 8/92/0 |
| AP cC No. 19 | 0/100/0 |
| AP cC No. 20 | 26/74/0 |
| AP cC No. 21 | 0/100/0 |
| AP cC No. 22 | 13/87/0 |
| AP cC No. 23 | 6/94/0 |
| AP cC No. 24 | 0/100/0 |
| AP cC No. 25 | 0/100/0 |
| AP cC No. 26 | 10/90/0 |
| AP cC No. 27 | 1/99/0 |
| AP cC No. 28 | 10/90/0 |
| AP cC No. 29 | 16/84/0 |
| AP cC No. 30 | 12/88/0 |
| AP cC No. 31 | 12/88/0 |
| AP cC No. 32 | 3/97/0 |
| AP cC No. 33 | 3/97/0 |
| AP cC No. 34 | 0/100/0 |
| AP cC No. 35 | 0/100/0 |
| AP cC No. 36 | 0/100/0 |
| AP cC No. 37 | 36/64/0 |
| AP cC No. 38 | 0/100/0 |
| AP cC No. 39 | 17/83/0 |
| AP cC No. 40 | 1/99/0 |
| AP cC No. 41 | 9/91/0 |
| AP cC No. 42 | 25/75/0 |

As shown in the following Tables 4-6, lily varieties Acapulco, R094-19, Tiara and MP8 were transformed using Agrobacterium containing pNARI-cTr3, and 123, 46, 33 and 2 cultivars of genetically modified plants were respectively acquired. The average delphinidin content in the petioles where anthocyanins had accumulated was 10%, 15%, 20% and 4%, respectively, and the maximum delphinidin content was 63% (individual no. AP cTr3 No. 80), 48%, 54% and 7%, respectively. The analysis results are summarized in the following Tables 4 to 6. When the genetically modified Acapulco varieties were allowed to bloom and analyzed for anthocyanidins in the petals thereof, the delphinidin content in a cultivar designated as No. 80-1 was 73%. The flower color thereof was similar to that of genetically modified Acapulco (described in Example 1) that demonstrated a delphinidin content of 80%.

**[Table 4]**

| Individual No. | HPLC Petiole (Del/Cya/Pel: %) |
|---|---|
| AP cTr3 No. 1 | 1/99/0 |
| AP cTr3 No. 2 | 21/75/4 |
| AP cTr3 No. 3 | 0/100/0 |
| AP cTr3 No. 4 | 3/97/0 |
| AP cTr3 No. 5 | 1/99/0 |
| AP cTr3 No. 6 | 8/91/1 |
| AP cTr3 No. 7 | 30/58/12 |
| AP cTr3 No. 8 | 29/71/0 |
| AP cTr3 No. 9 | 30/67/3 |
| AP cTr3 No. 10 | 0/100/0 |
| AP cTr3 No. 11 | 18/79/3 |
| AP cTr3 No. 12 | 5/94/1 |
| AP cTr3 No. 13 | 7/93/0 |
| AP cTr3 No. 14 | 12/86/2 |
| AP cTr3 No. 15 | 18/78/4 |
| AP cTr3 No. 16 | 6/94/0 |
| AP cTr3 No. 17 | 0/100/0 |
| AP cTr3 No. 18 | 15/85/0 |
| AP cTr3 No. 19 | 39/38/23 |
| AP cTr3 No. 20 | 1/99/0 |
| AP cTr3 No. 21 | 1/99/0 |
| AP cTr3 No. 22 | 9/91/0 |
| AP cTr3 No. 23 | 23/75/2 |
| AP cTr3 No. 24 | 1/99/0 |
| AP cTr3 No. 25 | 2/98/0 |
| AP cTr3 No. 26 | 43/56/1 |
| AP cTr3 No. 27 | 31/66/3 |
| AP cTr3 No. 28 | 1/99/0 |
| AP cTr3 No. 29 | 1/99/0 |
| AP cTr3 No. 30 | 1/99/0 |
| AP cTr3 No. 31 | 1/99/0 |
| AP cTr3 No. 32 | 23/75/2 |
| AP cTr3 No. 33 | 2/97/1 |
| AP cTr3 No. 34 | 14/84/2 |
| AP cTr3 No. 35 | 1/99/0 |
| AP cTr3 No. 36 | 1/99/0 |
| AP cTr3 No. 37 | 1/99/1 |
| AP cTr3 No. 38 | 1/99/0 |

**[Table 5]**

| Individual No. | HPLC Petiole (Del/Cya/Pel: %) |
|---|---|
| AP cTr3 No. 39 | 0/100/0 |
| AP cTr3 No. 40 | 15/85/0 |
| AP cTr3 No. 41 | 4/96/0 |
| AP cTr3 No. 42 | 3/97/0 |
| AP cTr3 No. 43 | 22/76/2 |
| AP cTr3 No. 44 | 2/98/0 |
| AP cTr3 No. 45 | 1/99/0 |
| AP cTr3 No. 46 | 10/78/12 |
| AP cTr3 No. 47 | 7/93/0 |
| AP cTr3 No. 48 | 3/97/0 |
| AP cTr3 No. 49 | 3/97/0 |
| AP cTr3 No. 50 | 5/95/0 |
| AP cTr3 No. 51 | 7/93/0 |
| AP cTr3 No. 52 | 10/90/0 |
| AP cTr3 No. 53 | 9/88/3 |
| AP cTr3 No. 54 | 9/91/0 |
| AP cTr3 No. 55 | 1/99/0 |
| AP cTr3 No. 56 | 28/72/0 |
| AP cTr3 No. 57 | 10/90/0 |
| AP cTr3 No. 58 | 0/100/0 |
| AP cTr3 No. 59 | 27/73/0 |
| AP cTr3 No. 60 | 3/97/0 |
| AP cTr3 No. 61 | 1/99/0 |
| AP cTr3 No. 62 | 20/80/0 |
| AP cTr3 No. 63 | 1/99/0 |
| AP cTr3 No. 64 | 39/55/6 |
| AP cTr3 No. 65 | 1/99/0 |
| AP cTr3 No. 66 | 6/94/0 |
| AP cTr3 No. 67 | 0/100/0 |
| AP cTr3 No. 68 | 6/92/2 |
| AP cTr3 No. 69 | 10/88/2 |
| AP cTr3 No. 70 | 9/91/0 |
| AP cTr3 No. 71 | 0/100/0 |
| AP cTr3 No. 72 | 3/97/0 |
| AP cTr3 No. 73 | 4/93/3 |
| AP cTr3 No. 74 | 1/98/1 |
| AP cTr3 No. 75 | 1/99/0 |
| AP cTr3 No. 76 | 0/100/0 |
| AP cTr3 No. 77 | 1/99/0 |
| AP cTr3 No. 78 | 18/82/0 |
| AP cTr3 No. 79 | 13/87/0 |
| AP cTr3 No. 80 | 63/31/6 |
| AP cTr3 No. 81 | 15/85/0 |
| AP cTr3 No. 82 | 0/100/0 |

**[Table 6]**

| Individual No. | HPLC Petiole (Del/Cya/Pel: %) | Del |
|---|---|---|
| AP cTr3 No. 83 | 7/93/0 | 7 |
| AP cTr3 No. 84 | 5/95/0 | 5 |
| AP cTr3 No. 85 | 16/84/0 | 16 |
| AP cTr3 No. 86 | 31/67/2 | 31 |
| AP cTr3 No. 87 | 8/92/0 | 8 |
| AP cTr3 No. 88 | 1/99/0 | 1 |
| AP cTr3 No. 89 | 19/81/0 | 19 |
| AP cTr3 No. 90 | 0/100/0 | 0 |
| AP cTr3 No. 91 | 0/100/0 | 0 |
| AP cTr3 No. 92 | 36/57/7 | 36 |
| AP cTr3 No. 93 | 49/20/31 | 49 |
| AP cTr3 No. 94 | 4/93/3 | 4 |
| AP cTr3 No. 95 | 1/99/0 | 1 |
| AP cTr3 No. 96 | 3/97/0 | 3 |
| AP cTr3 No. 97 | 0/100/0 | 0 |
| AP cTr3 No. 98 | 25/65/10 | 25 |
| AP cTr3 No. 99 | 1/99/0 | 1 |
| AP cTr3 No. 100 | 6/94/0 | 6 |
| AP cTr3 No. 101 | 17/83/0 | 17 |
| AP cTr3 No. 102 | 1/98/1 | 1 |
| AP cTr3 No. 103 | 1/99/0 | 1 |
| AP cTr3 No. 104 | 20/80/0 | 20 |
| AP cTr3 No. 105 | 1/99/0 | 1 |
| AP cTr3 No. 106 | 8/92/0 | 8 |
| AP cTr3 No. 107 | 0/100/0 | 0 |
| AP cTr3 No. 108 | 2/98/0 | 2 |
| AP cTr3 No. 109 | 0/100/0 | 0 |
| AP cTr3 No. 110 | 2/98/0 | 2 |
| AP cTr3 No. 111 | 13/87/0 | 13 |
| AP cTr3 No. 112 | 45/54/1 | 45 |
| AP cTr3 No. 113 | 54/41/5 | 54 |
| AP cTr3 No. 114 | 2/98/0 | 2 |
| AP cTr3 No. 115 | 16/84/0 | 16 |
| AP cTr3 No. 116 | 2/98/0 | 2 |
| AP cTr3 No. 117 | 1/99/0 | 1 |
| AP cTr3 No. 118 | 0/100/0 | 0 |
| AP cTr3 No. 119 | 0/100/0 | 0 |
| AP cTr3 No. 120 | 25/72/3 | 25 |
| AP cTr3 No. 121 | 1/99/0 | 1 |
| AP cTr3 No. 122 | 16/84/0 | 16 |
| AP cTr3 No. 123 | 5/95/0 | 5 |
| Average | | 10.05 |

### [Example 3: Tr3M Construction and Lily Transformation]

The terminator of campanula F3'5'H gene contained in the T-DNA of pNARI-Tr3 was changed from the terminator derived from nopaline synthase gene to a terminator derived from manopine synthase gene (Patent Document 1). The resulting vector was designated as pTr3M. Lily varieties Acapulco, R094-19, Tiara and MP8 were transformed using Agrobacterium containing this vector, and 6, 19, 71 and 11 cultivars, respectively, of genetically modified plants were acquired. The average delphinidin content of petioles where anthocyanins had accumulated was 38%, 34%, 7% and 22%, respectively, and the maximum delphinidin content was 72%, 70%, 54% and 65%, respectively.

### [Example 4: Tr7 Construction and Lily Transformation]

### (1) Acquisition of Dihydroflavonol 4-Reductase (DFR) Gene from Lilies

A cDNA library derived from the petals of lily variety Aktiva (Yamaki Noen Co., Ltd.) was prepared by digesting Lambda ZAP II vector with EcoRI and XhoI, and using the dephosphorylated Uni-ZAP XR vector for directional cloning (Agilent Technologies Inc.). The method described in WO 01/092509, for example, can be used to prepare the cDNA library. A plasmid pSPB3165 containing lily DFR cDNA (SEQ ID NO: 13) between the EcoRI and XhoI restriction enzyme sites of pBluescriptII vector was acquired by screening the resulting cDNA library with labeled geranium DFR cDNA (GenBank Accession No. AB534774, described in Plant Biotechnology (2010) 27, 375-383). Screening of the cDNA library can be carried out according to, for example, the method described in WO 81/092509.

### (2) Acquisition of Lily F3'H cDNA

The aforementioned lily petal cDNA library was screened using the F3'H DNA fragment contained in pCR2.1-AP24-1 described in Example 1. The plasmid containing F3'H cDNA was designated as pSBP3175. Since this cDNA was not full length, the missing sequence on the 5'-side was amplified using the Gene Racer Kit (Invitrogen Corp.) according to the method recommended by the manufacturer. The synthetic oligonucleotide LiRG-R3: 5'-TGCTCCGCTATCAACTTATC-3' (SEQ ID NO: 15) was used for the PCR reaction. The resulting DNA fragment was cloned in pCR-TOPOII to obtain plasmid pSPB3194. An approximately 700 bp DNA fragment obtained from this plasmid by digesting with PstI and SspI was inserted between the PstI and SspI sites of plasmid pSPB3175 to obtain plasmid pSPB3198. The synthesized lily F3'H cDNA (SEQ ID NO: 16) is contained in this plasmid.

### (3) Vector Construction

A binary vector Tr7 for suppressing expression of lily F3'H gene and DFR gene and expressing campanula F3'5'H gene and iris DFR gene in lilies was constructed.

A cassette for suppressing expression of lily DFR gene was constructed in the manner described below. An approximately 0.5 kp DNA fragment obtained by digesting plasmid pSPB3165 with EcoRI and PstI (containing a sequence encoding the vicinity of the N-terminal lily DFR cDNA) was cloned between the EcoRI and PstI sites of the plasmid pBluescriptSK- (Agilent Technologies Inc.). Plasmid pSPB3168 was obtained by ligating an approximately 0.5 kb DNA fragment obtained by digesting this plasmid with BamHI and SalI (containing a sequence encoding the vicinity of the N-terminal of lily DFR cDNA), an approximately 3.5 kb DNA fragment obtained by digesting plasmid pSPB906 (described in WO 2005/017147) with BamHI and EcoRI, and an approximately 300 bp DNA fragment obtained by digesting plasmid pSPB176 with SalI and EcoRI (containing a nopaline synthase terminator). Plasmid pSPB3178 was obtained by ligating an approximately 0.8 kb DNA fragment obtained by digesting the aforementioned plasmid pSPB3165 with BamHI and EcoRV (containing a sequence encoding a portion on the N-terminal side of lily DFR cDNA) with plasmid pSPB3168 digested with BamHI and SmaI. This plasmid contains a cassette for allowing 35S promoter to which an enhancer has been added to constitutively transcribe double-stranded RNA of lily DFR cDNA. A plasmid in which the 35S promoter to which an enhancer of plasmid pSPB3178 had been added was replaced with a 35S promoter sequence was designated as pSPB3456.

A plasmid in which a 35S promoter sequence to which an enhancer of plasmid pSPB912 (described in WO 2005/017147) had been added was replaced with 35S promoter sequence was designated as plasmid pSPB3419. This plasmid is used for the purpose of constitutively expressing iris DFR cDNA. A cassette for suppressing expression of lily F3'H gene was prepared in the manner described below. After digesting pSPB3198 with HaeII, it was further digested with BamHI followed by recovery of a DNA fragment containing the 5'-side of F3'H cDNA. Next, pSPB3194 was digested with HaeII followed by blunting, further digesting with XhoI and recovering a DNA fragment containing the 5'-side of F3'H cDNA. These DNA fragments were ligated with pSPB912 digested with BamHI and SalI to obtain plasmid pSPB3200. A plasmid in which a 35S promoter sequence to which an enhancer of pSBP3200 had been added was replaced with 35S promoter sequence was designated as plasmid pSPB3455. Transcription cassettes were respectively recovered from pSPB3456, pSPB3419 and pSPB3455 and used to construct Tr7.

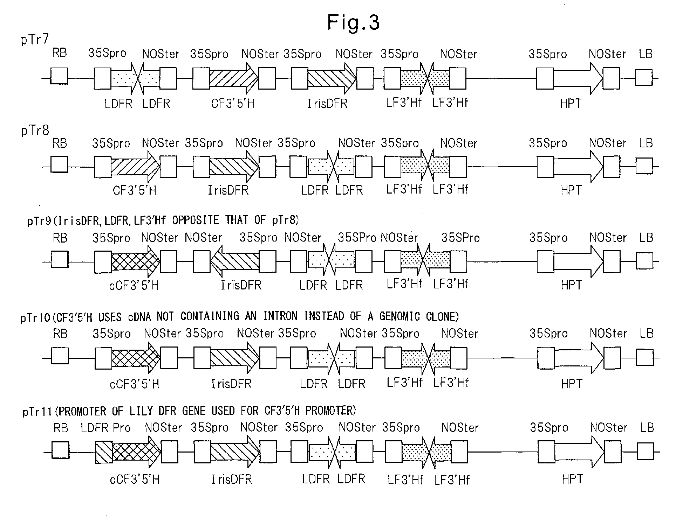

Tr7 contains five transcription cassettes on T-DNA consisting of, when moving from the right border sequence side, (i) 35S promoter sequence, sequence for transcribing double-stranded RNA of lily DFR cDNA and nopaline synthase terminator, (ii) 35S promoter sequence, campanula F3'5'H gene sequence containing an intron, and nopaline synthase terminator (same as that contained in Tr3), (iii) 35S promoter sequence, iris DFR gene sequence and nopaline synthase terminator, (iv) 35S promoter sequence, sequence for transcribing double-stranded RNA of lily F3'H cDNA and nopaline synthase terminator (same as contained in Tr3), and (v) 35S promoter sequence, hygromycin phosphotransferase gene sequence, and nopaline synthase terminator (same as contained in Tr3). A map of Tr7 is shown in FIG. 3.

### (4) Preparation of Genetically Modified Lilies

Lily varieties Acapulco, R094-19, Tiara and MP8 were transformed using Agrobacterium containing Tr7, and 22, 80, 32 and 11 cultivars, respectively, of genetically modified plants were acquired. The average delphinidin content of petioles in which anthocyanins accumulated was 15%, 13%, 6% and 28%, respectively, and the maximum delphinidin content was 78%, 88%, 56% and 87%, respectively.

The R094 genetically modified cultivar R094-19 Tr7 No. 52-3 had a petal delphinidin content of 99%.

### [Example 5: Tr8 Construction and Lily Transformation]

Binary vector Tr8 was prepared in which the order of the expression cassettes on T-DNA differs from that of binary vector Tr7. Tr8 contains five transcription cassettes on T-DNA consisting of, when moving from the right border sequence side, (i) 35S promoter sequence, campanula F3'5'H gene sequence containing an intron, and nopaline synthase terminator (same as that contained in Tr3), (ii) 35S promoter sequence, iris DFR gene sequence and nopaline synthase terminator, (iii) 35S promoter sequence, sequence for transcribing double-stranded RNA of lily DFR cDNA, and nopaline synthase terminator (same as that contained in Tr7), (iv) 35S promoter sequence, sequence for transcribing double-stranded RNA of lily F3'H cDNA and nopaline synthase terminator (same as contained in Tr7), and (v) 35S promoter sequence, hygromycin phosphotransferase gene sequence, and nopaline synthase terminator (same as contained in Tr3). A map of Tr8 is shown in FIG. 3. Lily varieties Acapulco, R094-19 and Tiara were transformed using Agrobacterium containing Tr8, and 66, 6 and 87 cultivars, respectively, of genetically modified plants were acquired. The average delphinidin content of petioles in which anthocyanins accumulated was 15%, 6% and 30%, respectively (the petioles of 8 of the 87 cultivars were analyzed), and the maximum delphinidin content was 51%, 6% and 70%, respectively.

### [Example 6: Tr9 Construction and Lily Transformation]

Binary vector Tr9 was constructed that expresses campanula F3'5'H cDNA. A map of Tr9 is shown in FIG. 3. Tr9 contains five transcription cassettes on T-DNA consisting of, when moving from the right border sequence side, (i) nopaline synthase terminator (same as that contained in cTr3), cDNA sequence of campanula F3'5'H gene, and 35S promoter sequence, (ii) 35S promoter sequence, sequence for transcribing double-stranded RNA of lily DFR cDNA, and nopaline synthase terminator (same as that contained in Tr7 but having a different orientation), (iii) nopaline synthase terminator, sequence for transcribing double-stranded RNA of lily F3'H cDNA, and 35S promoter sequence (same as that contained in Tr7 but having a different orientation), (iv) nopaline synthase terminator, sequence for transcribing double-stranded RNA of lily F3'H cDNA (same as that contained in Tr7 but having a different orientation), and (v) 35S promoter sequence, hygromycin phosphotransferase gene sequence, and nopaline synthase terminator (same as contained in Tr3). Lily varieties Acapulco, R094-19 and Tiara were transformed using Agrobacterium containing Tr9, and 8, 51 and 33 cultivars, respectively, of genetically modified plants were acquired. The average delphinidin content of petioles in which anthocyanins accumulated was 2%, 12% (the petioles of 44 of the 51 cultivars were analyzed) and 13%, respectively, and the maximum delphinidin content was 9%, 68% and 38%, respectively.

### [Example 7: Construction of Tr10 and Lily Transformation]

Tr10 contains five transcription cassettes on T-DNA consisting of, when moving from the right border sequence side, (i) 35S promoter sequence, cDNA sequence of campanula F3'5'H gene, and nopaline synthase terminator (same as that contained in cTr3), (ii) 35S promoter sequence, iris DFR gene sequence, and nopaline synthase terminator (same as that contained in Tr7), (iii) 35S promoter sequence, sequence for transcribing double-stranded RNA of lily DFR cDNA, and nopaline synthase terminator (same as that contained in Tr7), (iv) 35S promoter sequence, sequence for transcribing double-stranded RNA of lily F3'H cDNA, and nopaline synthase terminator (same as that contained in Tr7), and (v) 35S promoter sequence, hygromycin phosphotransferase gene sequence, and nopaline synthase terminator (same as contained in Tr3). A map of Tr10 is shown in FIG. 3. Lily varieties Acapulco, R094-19, Tiara and MP8 were transformed using Agrobacterium containing Tr10, and 41, 14, 78 and 10 cultivars, respectively, of genetically modified plants were acquired. The average delphinidin content of petioles in which anthocyanins accumulated was 23%, 26%, 15% (the petioles of 19 cultivars were analyzed) and 36%, respectively, and the maximum delphinidin content was 80%, 87%, 75% and 74%, respectively.

The flower color of the genetically modified Acapulco cultivar AP Tr10 No. 34-1 changed distinctly (color chart no. N78A), and the delphinidin content in the petals was 82%.

### [Example 8: Construction of Tr11 and Lily Transformation]

### (1) Isolation of Promoter Sequence of Lily DFR Gene

Isolation of the promoter sequence of lily DFR gene was carried out by inverse PCR. Four primers consisting of cLD2L: 5'-AGGGTCTCTCACAGTAGCCCGAACCGTGTAGCCA-3' (SEQ ID NO: 18), LD1L: 5'-ACTAGATCTTCCTGGAGCTGATGAACGGCTAACC-3' (SEQ ID NO: 19), cLD1L: 5'-AACATAGCCACTAGCTCCAGTAACCACCACGGGTC-3' (SEQ ID NO: 20) and LD3L: 5'-AGCAAATCCAGCTTGTTCACTTGGATGACGTATGC-3' (SEQ ID NO: 21) were synthesized based on the DFR cDNA sequence of the lily variety Montreux (pink flowers) registered in the GenBank DNA database (DNA Database Accession No. AB058641). DNA was purified from the leaves of the lily variety Montreux (Yamaki Noen Co., Ltd.) using the DNeasy Plant Mini Kit (Qiagen Corp.). After cleaving the resulting DNA with restriction enzyme EcoRI, cyclic DNA was prepared by a self-ligation reaction of the DNA fragments using a DNA Ligation Kit (Mighty Mix, Takara Bio Inc.). A first round of a PCR reaction was carried out using the Takara LA Taq Hot Start Version (Takara Bio Inc.) by using this cyclic DNA as template and using cLD2L and LD1L as primers. Moreover, a second round of a PCR reaction was carried out using the Takara LA Taq Hot Start Version by using the amplified DNA as template and using cLD1L and LD3L as primers to obtain a DNA fragment containing the promoter portion of DFR. The resulting DNA fragment was ligated to plasmid pCR2.1 (Invitrogen Corp.). The resulting plasmid was designated as pCR-LDE. Moreover, the promoter sequence of lily DFR gene (SEQ ID NO: 24) was amplified using this plasmid as template, and using the synthetic nucleotides AscI-HindIII-LDE6-F (5'-GGCGCGCCAAGCTTGAATTCTTAGCGGAAATTATCC-3' (SEQ ID NO: 22) and XbaI-LDE6-R (5'-TCTAGAATCTCTCTCTCTCCCCCTC-3' (SEQ ID NO: 23) as primers. A DNA fragment containing this sequence can be recovered by digesting with HindIII and XbaI.

### (2) Vector Construction and Lily Transformation

Tr11 contains five transcription cassettes on T-DNA consisting of, when moving from the right border sequence side, (i) lily DFR promoter sequence, campanula F3'5'H gene cDNA sequence, and nopaline synthase terminator, (ii) 35S promoter sequence, iris DFR gene sequence, and nopaline synthase terminator (same as that contained in Tr7), (iii) 35S promoter sequence, sequence for transcribing double-stranded RNA of lily DFR cDNA, and nopaline synthase terminator (same as that contained in Tr7), (iv) 35S promoter sequence, sequence for transcribing double-stranded RNA of lily F3'H cDNA, and nopaline synthase terminator (same as that contained in Tr7), and (v) 35S promoter sequence, hygromycin phosphotransferase gene sequence, and nopaline synthase terminator (same as contained in Tr3). A map of Tr11 is shown in FIG. 3. Lily varieties R094-19, Tiara and MP8 were transformed using Agrobacterium containing Tr11, and 66, 120 and 18 cultivars, respectively, of genetically modified plants were acquired. The average delphinidin content of petioles in which anthocyanins accumulated was 40%, 14% and 38%, respectively, and the maximum delphinidin content was 100% (52 cultivars analyzed), 54% (14 cultivars analyzed) and 92% (11 cultivars analyzed), respectively.

### INDUSTRIAL APPLICABILITY

According to the present invention, delphinidin not found in nature can be produced and lilies in which flower color has changed to blue can be produced by suppressing expression of one or multiple endogenous flavonoid synthase genes in lilies, and expressing F3'5'H gene or F3'5'H gene and DFR gene. Thus, the present invention can be preferably used in such industries as the flowering plant industry.

### BRIEF DESCRIPTION OF THE REFERENCE SYMBOLS

RB Right border
LB Left border
35Spro Cauliflower mosaic virus 35S promoter
NOSter Nopaline synthase terminator
LF3'hf Lily F3'H gene fragment
CF3'5'H Campanula F3'5'H gene
IrisDFR Iris DFR gene
cCF3'5'H Campanula F3'5'H cDNA
LDFR Lily DFR
HPT Hygromycin resistance gene

## Claims

1. A method for producing lilies containing delphinidin in the petals thereof, comprising the following steps:
introducing into lilies a campanula-derived flavonoid 3',5'-hydroxylase (F3'5'H) gene composed of a nucleotide sequence represented by SEQ ID NO: 1 or SEQ ID NO: 11, or a nucleotide sequence having at least 90% sequence identity with the nucleotide sequence represented by SEQ ID NO: 1 or SEQ ID NO: 11 that also encodes a polypeptide having F3'5'H activity,
introducing a fragment of lily-derived flavonoid 3'-hydroxylase (F3'H) gene composed of a nucleotide sequence represented by SEQ ID NO: 3 or SEQ ID NO: 16, or a nucleotide sequence having at least 90% sequence identity with the nucleotide sequence represented by SEQ ID NO: 3 or SEQ ID NO: 16 that also encodes a polypeptide having F3'H activity, and
introducing a fragment of lily-derived dihydroflavonol 4-reductase (DFR) gene composed of a nucleotide sequence represented by SEQ ID NO: 13 or a nucleotide sequence having at least 90% sequence identity with the nucleotide sequence represented by SEQ ID NO: 13 that also encodes a polypeptide having DFR activity, to suppress expression of endogenous DFR that acts on cyanidin synthesis in lily petals, and simultaneously introducing an iris-derived foreign DFR gene;
synthesizing delphinidin by the action of the introduced F3'5'H gene while suppressing expression of endogenous F3'H gene that acts on cyanidin synthesis in lily petals.

2. The method according to claim 1, wherein the suppression of expression of endogenous F3'H is carried out in the RNAi method.

3. The method according to claim 1 or claim 2, wherein the campanula-derived F3'5'H gene is composed of a nucleotide sequence having at least 95% sequence identity with the nucleotide sequence represented by SEQ ID NO: 1 or SEQ ID NO: 11 that also encodes a polypeptide having F3'5'H activity.

4. The method according to claim 3, wherein the campanula-derived F3'5'H gene is composed of a nucleotide sequence represented by SEQ ID NO: 1 or SEQ ID NO: 11.

5. The method according to claim 1 or claim 2, wherein the fragment of lily-derived F3'H gene is composed of a nucleotide sequence having at least 95% sequence identity with the nucleotide sequence represented by SEQ ID NO: 3 or SEQ ID NO: 16 that also encodes a polypeptide having F3'H activity.

6. The method according to claim 5, wherein the fragment of lily-derived F3'H gene is composed of a nucleotide sequence represented by SEQ ID NO: 3 or SEQ ID NO: 16.

7. The method according to any one of claims 1 to 6, further comprising: using a lily DFR promoter sequence represented by SEQ ID NO: 24.

8. A lily, tissue thereof, portion thereof, vegetatively propagated plant thereof or progeny thereof, which contains delphinidin in the petals thereof produced according to the method according to any of the preceding claims.

9. The lily, tissue thereof, portion thereof, vegetatively propagated plant thereof or progeny thereof according to claim 8, wherein the tissue or portion thereof is a cut flower.

## Patentansprüche

1. Verfahren zur Herstellung von Lilien, die Delphinidin in den Blütenblättern enthalten, das folgende Schritte umfasst:
Einbringen eines von einer Glockenblume stammenden Flavonoid-3',5'-hydroxylase- (F3'5'H-) Gens, das aus einer Nucleotidsequenz der Seq.-ID Nr. 1 oder Seq.-ID Nr. 11 oder einer Nucleotidsequenz mit zumindest 90 % Sequenzidentität mit der Nucleotidsequenz der Seq.-ID Nr. 1 oder Seq.-ID Nr. 11 besteht, die auch für ein Polypeptid mit F3'5'H-Aktivität kodiert, in Lilien,
Einbringen eines Fragments eines von einer Lilie stammenden Flavonoid-3'-Hydroxylase- (F3'H-) Gens, das aus einer Nucleotidsequenz der Seq.-ID Nr. 3 oder Seq.-ID Nr. 16 oder einer Nucleotidsequenz mit zumindest 90 % Sequenzidentität mit der Nucleotidsequenz der Seq.-ID Nr. 3 oder Seq.-ID Nr. 16 besteht, die auch für ein Polypeptid mit F3'H-Aktivität kodiert, und
Einbringen eines Fragments eines von einer Lilie stammenden Dihydroflavonol-4-reductase (DFR-) Gens, das aus einer Nucleotidsequenz der Seq.-ID Nr. 13 oder einer Nucleotidsequenz mit zumindest 90 % Sequenzidentität mit der Seq.-ID Nr. 13 besteht, die auch für ein Polypeptid mit DFR-Aktivität kodiert, um die Expression von endogenem DFR zu unterdrücken, das in Lilienblütenblättern auf die Cyanidinsynthese wirkt, und gleichzeitiges Einbringen eines von einer Schwertlilie stammenden, fremden DFR-Gens;
Synthetisieren von Delphinidin durch die Wirkung des eingeführten F3'5'H-Gens, während gleichzeitig die Expression von endogenem F3'H-Gen, das in Lilienblütenblättern auf die Cyanidinsynthese wirkt, unterdrückt wird.

2. Verfahren nach Anspruch 1, wobei das Unterdrücken der Expression von endogenem F3'H im RNAi-Verfahren ausgeführt wird.

3. Verfahren nach Anspruch 1 oder Anspruch 2, wobei das von einer Glockenblume stammende F3'5'H-Gen aus einer Nucleotidsequenz mit zumindest 95 % Sequenzidentität mit der Nucleotidsequenz der Seq.-ID Nr. 1 oder Seq.-ID Nr. 11 besteht, die auch für ein Polypeptid mit F3'5'H-Aktivität kodiert.

4. Verfahren nach Anspruch 3, wobei das von einer Glockenblume stammende F3'5'H-Gen aus einer Nucleotidsequenz der Seq.-ID Nr. 1 oder Seq.-ID Nr. 11 besteht.

5. Verfahren nach Anspruch 1 oder Anspruch 2, wobei das Fragment eines von einer Lilie stammenden F3'H-Gens aus einer Nucleotidsequenz mit zumindest 95 % Sequenzidentität mit der Nucleotidsequenz der Seq.-ID Nr. 3 oder Seq.-ID Nr. 16 besteht, die auch für ein Polypeptid mit F3'H-Aktivität kodiert.

6. Verfahren nach Anspruch 5, wobei das Fragment eines von einer Lilie stammenden F3'H-Gens aus einer Nucleotidsequenz der Seq.-ID Nr. 3 oder Seq.-ID Nr. 16 besteht.

7. Verfahren nach einem der Ansprüche 1 bis 6, das weiters Folgendes umfasst: Verwendung einer Lilien-DFR-Promotorsequenz der Seq.-ID Nr. 24.

8. Lilie, Gewebe davon, Teil davon, vegetativ vermehrte Pflanze davon oder Nachkomme davon, die/das/der Delphinidin in den Blütenblättern enthält und gemäß einem Verfahren nach einem der vorangegangenen Ansprüche hergestellt ist.

9. Lilie, Gewebe davon, Teil davon, vegetativ vermehrte Pflanze davon oder Nachkomme davon nach Anspruch 8, wobei das Gewebe oder der Teil davon eine Schnittblume ist.

## Revendications

1. Procédé de production de lys contenant des delphinidines dans leurs pétales, comprenant les étapes suivantes :
introduire dans les lys un gène de flavonoïde 3',5'-hydroxylase dérivé de campanule (F3'5'H) constitué d'une séquence de nucléotides représentée par la SEQ ID NO :1 ou la SEQ ID NO :11, ou une séquence de nucléotides ayant au moins 90% d'identité de séquence avec la séquence de nucléotides représentée par la SEQ ID NO :1 ou la SEQ ID NO :11 qui code également pour un polypeptide ayant une activité de F3'5'H,
introduire un fragment du gène de flavonoïde 3'-hydroxylase dérivé de lis (F3'H) constitué d'une séquence de nucléotides représentée par la SEQ ID NO :3 ou la SEQ ID NO :16, ou une séquence de nucléotides ayant au moins 90% d'identité de séquence avec la séquence de nucléotides représentée par la SEQ ID NO :3 ou la SEQ ID NO : :16 qui code également pour un polypeptide ayant une activité de F3'H, et
introduire un fragment du gène de di-hydro-flavonol 4-réductase dérivé de lys (DFR) constitué d'une séquence de nucléotides représentée par la SEQ ID NO :13 ou une séquence de nucléotides ayant au moins 90% d'identité de séquence avec la séquence de nucléotides représentée par la SEQ ID NO :13 qui code également pour un polypeptide ayant une activité de DFR, pour supprimer l'expression du DFR endogène qui agit sur une synthèse de la cyanidine dans les pétales de lys, et introduire simultanément un gène DFR étranger dérivé de l'iris ;
synthétiser la delphinidine par l'action du gène F3'5'H introduit tout en supprimer l'expression du gène F3'H endogène qui agit sur la synthèse de la cyanidine dans les pétales du lys.

2. Procédé selon la revendication 1, dans lequel la suppression de l'expression du F3'H endogène est exécutée dans la méthode ARNi.

3. Procédé selon la revendication 1 ou la revendication 2, dans lequel le gène F3'5'H dérivé de la campanule est constitué d'une séquence de nucléotides ayant au moins 95% d'identité de séquence avec la séquence de nucléotides représentée par la SEQ ID NO :1 ou la SEQ ID NO :11 qui code également pour un polypeptide ayant l'activité de F3'5'H.

4. Procédé selon la revendication 3, dans lequel le gène F3'5'H dérivé de la campanule est constitué d'une séquence de nucléotides représentée par la SEQ ID NO :1 ou la SEQ ID NO :11.

5. Procédé selon la revendication 1 ou la revendication 2, dans lequel le fragment du gène F3'H dérivé du lys est constitué d'une séquence de nucléotides ayant au moins 95% d'identité de séquence avec la séquence de nucléotides représentée par la SEQ ID NO :3 ou la SEQ ID NO :16 qui code également pour un polypeptide ayant l'activité F3'H.

6. Procédé selon la revendication 5, dans lequel le fragment du gène F3'H dérivé du lys est constitué d'une séquence de nucléotides représentée par la SEQ ID NO :3 ou la SEQ ID NO :16.

7. Procédé selon l'une quelconque des revendications 1 à 6, comprenant en outre : utiliser une séquence promoteur de DFR de lys représentée par la SEQ ID NO :24.

8. Lys, tissu de celui-ci, portion de celui-ci, plante végétativement propagée de celui-ci ou progéniture de celui-ci, qui contient de la delphinidine dans ses pétales produite en accord avec le procédé selon l'une quelconque des revendications précédentes.

9. Lys, tissu de celui-ci, portion de celui-ci, plante végétativement propagée de celui-ci ou progéniture de celui-ci en accord avec la revendication 8, où le tissu ou la portion de celui-ci est une fleur coupée.


### SEQUENCE LISTING

<110> Niigata Prefecture & Suntory Holdings Limited
<120> Process for the preparation of lily containing delphinidin in petals
<130> Z758-PCT
<150> JP2010-209349
   <151> 2010-09-17
<160> 24
<170> PatentIn version 3.5
<210> 1
   <211> 1731
   <212> DNA
   <213> Campanula medium
<220>
   <221> CDS
   <222> (2)..(940)
   <223> F3'5'H derived from Campanula genome DNA
<220>
   <221> CDS
   <222> (1097)..(1726)
<400> 1
   c atg tct ata gac ata tcc acc ctc ttc tat gaa ctt gtt gca gca att        49
     Met Ser Ile Asp Ile Ser Thr Leu Phe Tyr Glu Leu Val Ala Ala Ile
     1               5                   10                  15
   tca ctc tac tta gct acc tac tct ttc att cgt ttc ctc ttc aaa ccc          97
   Ser Leu Tyr Leu Ala Thr Tyr Ser Phe Ile Arg Phe Leu Phe Lys Pro
               20                  25                  30
   tct cac cac cac cac ctc cct ccc ggc cca acc gga tgg ccg atc atc         145
   Ser His His His His Leu Pro Pro Gly Pro Thr Gly Trp Pro Ile Ile
           35                  40                  45
   gga gcc ctt cca ctc tta ggc acc atg cca cat gtt tcc tta gcc gac         193
   Gly Ala Leu Pro Leu Leu Gly Thr Met Pro His Val Ser Leu Ala Asp
       50                  55                  60
   atg gcc gtt aaa tac ggt cct ata atg tac cta aaa ctt ggt tca aag         241
   Met Ala Val Lys Tyr Gly Pro Ile Met Tyr Leu Lys Leu Gly Ser Lys
   65                  70                  75                  80
   ggc acc gtc gtg gcc tca aat cca aaa gcc gcc cga gcc ttc ttg aaa         289
   Gly Thr Val Val Ala Ser Asn Pro Lys Ala Ala Arg Ala Phe Leu Lys
                   85                  90                  95
   acc cat gat gcc aat ttt tct aac cgt ccg att gat ggg ggc cct acc         337
   Thr His Asp Ala Asn Phe Ser Asn Arg Pro Ile Asp Gly Gly Pro Thr
               100                 105                 110
   tac ctc gcg tat aat gca caa gac atg gtt ttt gca gaa tat ggc cca         385
   Tyr Leu Ala Tyr Asn Ala Gln Asp Met Val Phe Ala Glu Tyr Gly Pro
           115                 120                 125
   aaa tgg aag ctt ttg cga aag cta tgt agc ttg cac atg tta ggc ccg        433
   Lys Trp Lys Leu Leu Arg Lys Leu Cys Ser Leu His Met Leu Gly Pro
       130                 135                 140
   aag gca ctc gag gat tgg gct cat gtc aaa gtt tca gag gtc ggt cat        481
   Lys Ala Leu Glu Asp Trp Ala His Val Lys Val Ser Glu Val Gly His
   145                 150                 155                 160
   atg ctc aaa gaa atg tac gag caa tcg agt aag tca gtg cca gtg cca        529
   Met Leu Lys Glu Met Tyr Glu Gln Ser Ser Lys Ser Val Pro Val Pro
                   165                 170                 175
   gtg gtg gtg cca gag atg tta act tat gcc atg gct aat atg att gga        577
   Val Val Val Pro Glu Met Leu Thr Tyr Ala Met Ala Asn Met Ile Gly
               180                 185                 190
   cga atc ata ctc agc cga cgc cct ttt gtt atc acg agc aaa tta gac        625
   Arg Ile Ile Leu Ser Arg Arg Pro Phe Val Ile Thr Ser Lys Leu Asp
           195                 200                 205
   tcg tct gct tct gct tct gct tct gtt agt gaa ttc caa tat atg gtt        673
   Ser Ser Ala Ser Ala Ser Ala Ser Val Ser Glu Phe Gln Tyr Met Val
       210                 215                 220
   atg gag ctc atg agg atg gca ggg ttg ttc aat att ggt gat ttc ata        721
   Met Glu Leu Met Arg Met Ala Gly Leu Phe Asn Ile Gly Asp Phe Ile
   225                 230                 235                 240
   cca tat att gca tgg atg gat ttg caa ggc att cag cgt gat atg aag        769
   Pro Tyr Ile Ala Trp Met Asp Leu Gln Gly Ile Gln Arg Asp Met Lys
                   245                 250                 255
   gtt ata cag aaa aag ttt gat gtc ttg ttg aac aaa atg atc aag gaa        817
   Val Ile Gln Lys Lys Phe Asp Val Leu Leu Asn Lys Met Ile Lys Glu
               260                 265                 270
   cat aca gaa tcc gct cat gat cgc aaa gat aat cct gat ttt ctt gat        865
   His Thr Glu Ser Ala His Asp Arg Lys Asp Asn Pro Asp Phe Leu Asp
           275                 280                 285
   att ctt atg gcg gct acc caa gaa aac acg gag gga att cag ctt aat        913
   Ile Leu Met Ala Ala Thr Gln Glu Asn Thr Glu Gly Ile Gln Leu Asn
       290                 295                 300
   ctt gta aat gtt aag gca ctt ctt ttg gtacgtatca tcaacttgga              960
   Leu Val Asn Val Lys Ala Leu Leu Leu
   305                 310
   tttgtatgaa catctaacat ttttctggaa aaagagctaa aaagggcact ctttaaaggg     1020
   tacttatttg tgacgaatat tatgcttgaa aatgaaatga atacttgttg aaattaatgc     1080
   tgtatattta tgctag gat tta ttc acg gcg ggc acg gat aca tca tca agt     1132
                     Asp Leu Phe Thr Ala Gly Thr Asp Thr Ser Ser Ser
                         315                 320                 325
   gtg atc gaa tgg gca cta gcc gaa atg ttg aac cat cga cag atc cta       1180
   Val Ile Glu Trp Ala Leu Ala Glu Met Leu Asn His Arg Gln Ile Leu
                   330                 335                 340
   aac cgg gcc cac gaa gaa atg gac caa gtc att ggc aga aac aga aga       1228
   Asn Arg Ala His Glu Glu Met Asp Gln Val Ile Gly Arg Asn Arg Arg
               345                 350                 355
   cta gaa caa tct gac ata cca aac ttg cca tat ttc caa gcc ata tgc       1276
   Leu Glu Gln Ser Asp Ile Pro Asn Leu Pro Tyr Phe Gln Ala Ile Cys
           360                 365                 370
   aaa gaa aca ttc cga aaa cac cct tcc acg ccc tta aac ctc cca aga       1324
   Lys Glu Thr Phe Arg Lys His Pro Ser Thr Pro Leu Asn Leu Pro Arg
       375                 380                 385
   atc tca aca gaa gca tgt gaa gtg gac gga ttt cac ata cca aaa aac       1372
   Ile Ser Thr Glu Ala Cys Glu Val Asp Gly Phe His Ile Pro Lys Asn
   390                 395                 400                 405
   act aga cta ata gtg aac ata tgg gca ata ggg agg gac cct aaa gtg       1420
   Thr Arg Leu Ile Val Asn Ile Trp Ala Ile Gly Arg Asp Pro Lys Val
                   410                 415                 420
   tgg gaa aat cca tta gat ttt act ccg gaa cgt ttc ttg agt gaa aaa       1468
   Trp Glu Asn Pro Leu Asp Phe Thr Pro Glu Arg Phe Leu Ser Glu Lys
               425                 430                 435
   cac gcg aaa att gac ccg cga ggt aat cat ttt gag tta atc cca ttt       1516
   His Ala Lys Ile Asp Pro Arg Gly Asn His Phe Glu Leu Ile Pro Phe
           440                 445                 450
   ggg gct gga cga agg ata tgt gca ggg gct aga atg gga gcg gcc tcg       1564
   Gly Ala Gly Arg Arg Ile Cys Ala Gly Ala Arg Met Gly Ala Ala Ser
       455                 460                 465
   gtc gag tac ata tta ggt aca ttg gtg cac tca ttt gat tgg aaa ttg       1612
   Val Glu Tyr Ile Leu Gly Thr Leu Val His Ser Phe Asp Trp Lys Leu
   470                 475                 480                 485
   cct gat gga gtt gtg gaa gtt aat atg gaa gag agc ttt ggg atc gca       1660
   Pro Asp Gly Val Val Glu Val Asn Met Glu Glu Ser Phe Gly Ile Ala
                   490                 495                 500
   ttg caa aaa aag gtg cct ctt tct gct att gtt act cca aga ttg cct       1708
   Leu Gln Lys Lys Val Pro Leu Ser Ala Ile Val Thr Pro Arg Leu Pro
               505                 510                 515
   cca agt tct tac act gtc taggc                                         1731
   Pro Ser Ser Tyr Thr Val
           520
<210> 2
   <211> 523
   <212> PRT
   <213> Campanula medium
<400> 2
   Met Ser Ile Asp Ile Ser Thr Leu Phe Tyr Glu Leu Val Ala Ala Ile
   1               5                   10                  15
   Ser Leu Tyr Leu Ala Thr Tyr Ser Phe Ile Arg Phe Leu Phe Lys Pro
               20                  25                  30
   Ser His His His His Leu Pro Pro Gly Pro Thr Gly Trp Pro Ile Ile
               35                  40                  45
   Gly Ala Leu Pro Leu Leu Gly Thr Met Pro His Val Ser Leu Ala Asp
       50                  55                  60
   Met Ala Val Lys Tyr Gly Pro Ile Met Tyr Leu Lys Leu Gly Ser Lys
   65                  70                  75                  80
   Gly Thr Val Val Ala Ser Asn Pro Lys Ala Ala Arg Ala Phe Leu Lys
                   85                  90                  95
   Thr His Asp Ala Asn Phe Ser Asn Arg Pro Ile Asp Gly Gly Pro Thr
               100                 105                 110
   Tyr Leu Ala Tyr Asn Ala Gln Asp Met Val Phe Ala Glu Tyr Gly Pro
           115                 120                 125
   Lys Trp Lys Leu Leu Arg Lys Leu Cys Ser Leu His Met Leu Gly Pro
       130                 135                 140
   Lys Ala Leu Glu Asp Trp Ala His Val Lys Val Ser Glu Val Gly His
   145                 150                 155                 160
   Met Leu Lys Glu Met Tyr Glu Gln Ser Ser Lys Ser Val Pro Val Pro
                   165                 170                 175
   Val Val Val Pro Glu Met Leu Thr Tyr Ala Met Ala Asn Met Ile Gly
               180                 185                 190
   Arg Ile Ile Leu Ser Arg Arg Pro Phe Val Ile Thr Ser Lys Leu Asp
           195                 200                 205
   Ser Ser Ala Ser Ala Ser Ala Ser Val Ser Glu Phe Gln Tyr Met Val
       210                 215                 220
   Met Glu Leu Met Arg Met Ala Gly Leu Phe Asn Ile Gly Asp Phe Ile
   225                 230                 235                 240
   Pro Tyr Ile Ala Trp Met Asp Leu Gln Gly Ile Gln Arg Asp Met Lys
                   245                 250                 255
   Val Ile Gln Lys Lys Phe Asp Val Leu Leu Asn Lys Met Ile Lys Glu
               260                 265                 270
   His Thr Glu Ser Ala His Asp Arg Lys Asp Asn Pro Asp Phe Leu Asp
           275                 280                 285
   Ile Leu Met Ala Ala Thr Gln Glu Asn Thr Glu Gly Ile Gln Leu Asn
       290                 295                 300
   Leu Val Asn Val Lys Ala Leu Leu Leu Asp Leu Phe Thr Ala Gly Thr
   305                 310                 315                 320
   Asp Thr Ser Ser Ser Val Ile Glu Trp Ala Leu Ala Glu Met Leu Asn
                   325                 330                 335
   His Arg Gln Ile Leu Asn Arg Ala His Glu Glu Met Asp Gln Val Ile
               340                 345                 350
   Gly Arg Asn Arg Arg Leu Glu Gln Ser Asp Ile Pro Asn Leu Pro Tyr
           355                 360                 365
   Phe Gln Ala Ile Cys Lys Glu Thr Phe Arg Lys His Pro Ser Thr Pro
       370                 375                 380
   Leu Asn Leu Pro Arg Ile Ser Thr Glu Ala Cys Glu Val Asp Gly Phe
   385                 390                 395                 400
   His Ile Pro Lys Asn Thr Arg Leu Ile Val Asn Ile Trp Ala Ile Gly
                   405                 410                 415
   Arg Asp Pro Lys Val Trp Glu Asn Pro Leu Asp Phe Thr Pro Glu Arg
               420                 425                 430
   Phe Leu Ser Glu Lys His Ala Lys Ile Asp Pro Arg Gly Asn His Phe
           435                 440                 445
   Glu Leu Ile Pro Phe Gly Ala Gly Arg Arg Ile Cys Ala Gly Ala Arg
       450                 455                 460
   Met Gly Ala Ala Ser Val Glu Tyr Ile Leu Gly Thr Leu Val His Ser
   465                 470                 475                 480
   Phe Asp Trp Lys Leu Pro Asp Gly Val Val Glu Val Asn Met Glu Glu
                   485                 490                 495
   Ser Phe Gly Ile Ala Leu Gln Lys Lys Val Pro Leu Ser Ala Ile Val
               500                 505                 510
   Thr Pro Arg Leu Pro Pro Ser Ser Tyr Thr Val
           515                 520
<210> 3
   <211> 575
   <212> DNA
   <213> Lilium sp.
<220>
   <221> CDS
   <222> (76)..(573)
   <223> cDNA of Lily F3'H
<400> 3
   ttggctggtg tttttaacat aggtgatttt gtgccggggc tgggttggtt ggatttgcag       60
   ggggtggtgg ggaag atg aag aag ttg cat aag agg atg gat gct ttt ctt       111
                    Met Lys Lys Leu His Lys Arg Met Asp Ala Phe Leu
                    1               5                   10
   gat aag ttg ata gcg gag cac cgg acg gca gcg gcc ggc ggc gag ttc       159
   Asp Lys Leu Ile Ala Glu His Arg Thr Ala Ala Ala Gly Gly Glu Phe
           15                  20                  25
   gcc gga aaa gat ttg ttg agt gtg atg gtt agg ttg cag gag gag gga       207
   Ala Gly Lys Asp Leu Leu Ser Val Met Val Arg Leu Gln Glu Glu Gly
       30                  35                  40
   gag ggg gag ggt ggg aaa ctg acc gac act gaa att aag gcg ctg tta       255
   Glu Gly Glu Gly Gly Lys Leu Thr Asp Thr Glu Ile Lys Ala Leu Leu
   45                  50                  55                  60
   ctg aac ttg ttc aca gca ggg aca gac acc acc tcc agc acc gta gag       303
   Leu Asn Leu Phe Thr Ala Gly Thr Asp Thr Thr Ser Ser Thr Val Glu
                   65                  70                  75
   tgg gct ctg gcc gag ctc atc cgt cac cct cca ctc ctc cac caa gct       351
   Trp Ala Leu Ala Glu Leu Ile Arg His Pro Pro Leu Leu His Gln Ala
               80                  85                  90
   cag aaa gag ctt gat act atc gtc ggt cac gac cgt ctc gtc tct gaa       399
   Gln Lys Glu Leu Asp Thr Ile Val Gly His Asp Arg Leu Val Ser Glu
           95                  100                 105
   tcc gac ctc ccc cac ctc ccc ttc cta agt gcc atc atc aaa gaa act       447
   Ser Asp Leu Pro His Leu Pro Phe Leu Ser Ala Ile Ile Lys Glu Thr
       110                 115                 120
   ttc cga ctc cac cca tcc acc cca ctc tca ctc cca cgg atg gcc tcc       495
   Phe Arg Leu His Pro Ser Thr Pro Leu Ser Leu Pro Arg Met Ala Ser
   125                 130                 135                 140
   gaa tca tgc acc atc aat ggc tac caa att cct aag aac gcc act cta       543
   Glu Ser Cys Thr Ile Asn Gly Tyr Gln Ile Pro Lys Asn Ala Thr Leu
                   145                 150                 155
   ctg gta aat gtg tgg gcc ata gcc cgt gac cc                            575
   Leu Val Asn Val Trp Ala Ile Ala Arg Asp
               160                 165
<210> 4
   <211> 166
   <212> PRT
   <213> Lilium sp.
<400> 4
   Met Lys Lys Leu His Lys Arg Met Asp Ala Phe Leu Asp Lys Leu Ile
   1               5                   10                  15
   Ala Glu His Arg Thr Ala Ala Ala Gly Gly Glu Phe Ala Gly Lys Asp
               20                  25                  30
   Leu Leu Ser Val Met Val Arg Leu Gln Glu Glu Gly Glu Gly Glu Gly
           35                  40                  45
   Gly Lys Leu Thr Asp Thr Glu Ile Lys Ala Leu Leu Leu Asn Leu Phe
       50                  55                  60
   Thr Ala Gly Thr Asp Thr Thr Ser Ser Thr Val Glu Trp Ala Leu Ala
   65                  70                  75                  80
   Glu Leu Ile Arg His Pro Pro Leu Leu His Gln Ala Gln Lys Glu Leu
                   85                  90                  95
   Asp Thr Ile Val Gly His Asp Arg Leu Val Ser Glu Ser Asp Leu Pro
               100                 105                 110
   His Leu Pro Phe Leu Ser Ala Ile Ile Lys Glu Thr Phe Arg Leu His
           115                 120                 125
   Pro Ser Thr Pro Leu Ser Leu Pro Arg Met Ala Ser Glu Ser Cys Thr
       130                 135                 140
   Ile Asn Gly Tyr Gln Ile Pro Lys Asn Ala Thr Leu Leu Val Asn Val
   145                 150                 155                 160
   Trp Ala Ile Ala Arg Asp
                   165
<210> 5
   <211> 35
   <212> DNA
   <213> Artificial
<220>
   <223> Nucleotide CFH1L
<400> 5
   catgtctata gacatatcca ccctcttcta tgaac 35
<210> 6
   <211> 35
   <212> DNA
   <213> Artificial
<220>
   <223> Nucleotide cCF1L
<400> 6
   gcctagacag tgtaagaact tggaggcaat cttgg 35
<210> 7
   <211> 23
   <212> DNA
   <213> Artificial
<220>
   <223> Oligonucleotide F3H2
<400> 7
   ytsgcyggwg twttyaacrt hgg 23
<210> 8
   <211> 20
   <212> DNA
   <213> Artificial
<220>
   <223> Olionucleotide cF3H4
<220>
   <221> misc_feature
   <222> (6)..(6)
   <223> n is a, c, g, or t
<400> 8
   ggrtcncgrg cwatggccca 20
<210> 9
   <211> 21
   <212> DNA
   <213> Artificial
<220>
   <223> Primer CamF1
<400> 9
   gtgaagccac catgtctata g 21
<210> 10
   <211> 22
   <212> DNA
   <213> Artificial
<220>
   <223> Primer CamR1
<400> 10
   gcatttgcct agacagtgta ag 22
<210> 11
   <211> 1585
   <212> DNA
   <213> Campanula medium
<220>
   <221> CDS
   <222> (12)..(1574)
<400> 11
   gtgaagccac c atg tct ata gac ata acc att ctc tta tgt gaa ctt gtt        50
                Met Ser Ile Asp Ile Thr Ile Leu Leu Cys Glu Leu Val
                1               5                   10
   gct gca att tca ctc tac tta tta acc tac tat ttc att tgt ttc ctc         98
   Ala Ala Ile Ser Leu Tyr Leu Leu Thr Tyr Tyr Phe Ile Cys Phe Leu
       15                  20                  25
   ttc aaa ccc tct cat cat cac cac cac ctc cct ccc ggc cca acc gga        146
   Phe Lys Pro Ser His His His His His Leu Pro Pro Gly Pro Thr Gly
   30                  35                  40                  45
   tgg ccg atc att gga tcc ctt cct ctc tta ggc act atg cca cat gtt        194
   Trp Pro Ile Ile Gly Ser Leu Pro Leu Leu Gly Thr Met Pro His Val
                   50                  55                  60
   tcc tta gcc gac atg gcc gta aaa tac ggg cct ata atg tac cta aaa        242
   Ser Leu Ala Asp Met Ala Val Lys Tyr Gly Pro Ile Met Tyr Leu Lys
               65                  70                  75
   ctt ggt tca aag ggc acc gtc gtg gcc tca aat cca aaa gcc gcc cga        290
   Leu Gly Ser Lys Gly Thr Val Val Ala Ser Asn Pro Lys Ala Ala Arg
           80                  85                  90
   gca ttc ttg aaa tcc cat gat gcc aat ttt tct aac cgt ccg att gat        338
   Ala Phe Leu Lys Ser His Asp Ala Asn Phe Ser Asn Arg Pro Ile Asp
       95                  100                 105
   ggg ggg ccc acc tac ctc gcg tat aat gca caa gac atg gtt ttt gca        386
   Gly Gly Pro Thr Tyr Leu Ala Tyr Asn Ala Gln Asp Met Val Phe Ala
   110                 115                 120                 125
   gaa tat ggc cca aaa tgg aag ctt ttg cga aag cta tgt agc ttg cac        434
   Glu Tyr Gly Pro Lys Trp Lys Leu Leu Arg Lys Leu Cys Ser Leu His
                   130                 135                 140
   atg tta ggc ccg aag gca ctc gag gat tgg gct cat gtc aga gtt tca        482
   Met Leu Gly Pro Lys Ala Leu Glu Asp Trp Ala His Val Arg Val Ser
               145                 150                 155
   gag gtc ggt cat atg ctc aaa gaa atg tac gag caa tcg agt aag tcc        530
   Glu Val Gly His Met Leu Lys Glu Met Tyr Glu Gln Ser Ser Lys Ser
           160                 165                 170
   gtg cca gtg gtg gtg cca gag atg tta act tat gcc atg gct aat atg        578
   Val Pro Val Val Val Pro Glu Met Leu Thr Tyr Ala Met Ala Asn Met
       175                 180                 185
   att gga cga atc ata ctc agt cga cgc cct ttt gtt atc acg agc aaa        626
   Ile Gly Arg Ile Ile Leu Ser Arg Arg Pro Phe Val Ile Thr Ser Lys
   190                 195                 200                 205
   tta gac tcg tct gct tct gct gct tct gtt agt gaa ttc caa tat atg        674
   Leu Asp Ser Ser Ala Ser Ala Ala Ser Val Ser Glu Phe Gln Tyr Met
                   210                 215                 220
   gtt atg gag ctc atg agg atg gca ggg ttg ttc aat att ggt gat ttc         722
   Val Met Glu Leu Met Arg Met Ala Gly Leu Phe Asn Ile Gly Asp Phe
               225                 230                 235
   ata cca tat att gcg tgg atg gat ttg caa ggc att caa cgc gat atg         770
   Ile Pro Tyr Ile Ala Trp Met Asp Leu Gln Gly Ile Gln Arg Asp Met
           240                 245                 250
   aag gtt ata cag caa aag ttt gat gtc ttg ttg aac aaa atg atc aag         818
   Lys Val Ile Gln Gln Lys Phe Asp Val Leu Leu Asn Lys Met Ile Lys     255
   260                 265
   gaa cat aca gaa tcc gct cat gat cgt aaa gat aat cct gat ttt ctt         866
   Glu His Thr Glu Ser Ala His Asp Arg Lys Asp Asn Pro Asp Phe Leu
   270                 275                 280                 285
   gat att ctt atg gcg gct acc caa gaa aac acg gag gga att cag ctt         914
   Asp Ile Leu Met Ala Ala Thr Gln Glu Asn Thr Glu Gly Ile Gln Leu
                   290                 295                 300
   aat ctc gta aat gtt aag gcg ctt ctt ttg gat tta ttc acg gcg ggc         962
   Asn Leu Val Asn Val Lys Ala Leu Leu Leu Asp Leu Phe Thr Ala Gly
               305                 310                 315
   acg gat aca tcg tcg agt gtg atc gaa tgg gca cta gcc gaa atg ttg         1010
   Thr Asp Thr Ser Ser Ser Val Ile Glu Trp Ala Leu Ala Glu Met Leu
           320                 325                 330
   aac aat cga cag atc cta aac cgg gcc cac gaa gaa atg gac caa gtc         1058
   Asn Asn Arg Gln Ile Leu Asn Arg Ala His Glu Glu Met Asp Gln Val
       335                 340                 345
   att ggc aga aac aga aga cta gaa caa tct gac ata cca aac ttg cca         1106
   Ile Gly Arg Asn Arg Arg Leu Glu Gln Ser Asp Ile Pro Asn Leu Pro
   350                 355                 360                 365
   tat ttc caa gcc ata tgc aaa gaa aca ttc cga aaa cac cct tcc acg         1154
   Tyr Phe Gln Ala Ile Cys Lys Glu Thr Phe Arg Lys His Pro Ser Thr
                   370                 375                 380
   ccc tta aac ctc cca aga atc tca aca gaa gaa tgt gaa gtc gaa gga         1202
   Pro Leu Asn Leu Pro Arg Ile Ser Thr Glu Glu Cys Glu Val Glu Gly
               385                 390                 395
   ttt cgc ata ccc aaa aac act aga cta ata gtg aac ata tgg gca ata         1250
   Phe Arg Ile Pro Lys Asn Thr Arg Leu Ile Val Asn Ile Trp Ala Ile
           400                 405                 410
   ggg aga gac cct aaa gtg tgg gaa aat cca ttg gat ttt acc ccg gaa         1298
   Gly Arg Asp Pro Lys Val Trp Glu Asn Pro Leu Asp Phe Thr Pro Glu
       415                 420                 425
   cga ttc ttg agt gaa aaa cac gcg aaa att gat ccg cga ggt aat cat         1346
   Arg Phe Leu Ser Glu Lys His Ala Lys Ile Asp Pro Arg Gly Asn His
   430                 435                 440                 445
   ttt gag tta atc cca ttt ggg gcg gga cgg agg ata tgt gca ggg gct         1394
   Phe Glu Leu Ile Pro Phe Gly Ala Gly Arg Arg Ile Cys Ala Gly Ala
                   450                 455                 460
   aga atg gga gcg gcc tcg gtc gag tac att tta ggt aca ttg gtg cac         1442
   Arg Met Gly Ala Ala Ser Val Glu Tyr Ile Leu Gly Thr Leu Val His
               465                 470                 475
   tca ttt gat tgg aaa ttg cct gat gga gtt gtg gaa gtt aat atg gaa       1490
   Ser Phe Asp Trp Lys Leu Pro Asp Gly Val Val Glu Val Asn Met Glu
           480                 485                 490
   gag agc ttt ggg ata gca ttg cag aaa aag atg cct ctt tct gct att       1538
   Glu Ser Phe Gly Ile Ala Leu Gln Lys Lys Met Pro Leu Ser Ala Ile
       495                 500                 505
   gtt act cca aga ttg cct cca agt gct tac act gtc taggcaaatg c          1585
   Val Thr Pro Arg Leu Pro Pro Ser Ala Tyr Thr Val
   510                 515                 520
<210> 12
   <211> 521
   <212> PRT
   <213> Campanula medium
<400> 12
   Met Ser Ile Asp Ile Thr Ile Leu Leu Cys Glu Leu Val Ala Ala Ile
   1               5                   10                  15
   Ser Leu Tyr Leu Leu Thr Tyr Tyr Phe Ile Cys Phe Leu Phe Lys Pro
               20                  25                  30
   Ser His His His His His Leu Pro Pro Gly Pro Thr Gly Trp Pro Ile
           35                  40                  45
   Ile Gly Ser Leu Pro Leu Leu Gly Thr Met Pro His Val Ser Leu Ala
       50                  55                  60
   Asp Met Ala Val Lys Tyr Gly Pro Ile Met Tyr Leu Lys Leu Gly Ser
   65                  70                  75                  80
   Lys Gly Thr Val Val Ala Ser Asn Pro Lys Ala Ala Arg Ala Phe Leu
                   85                  90                  95
   Lys Ser His Asp Ala Asn Phe Ser Asn Arg Pro Ile Asp Gly Gly Pro
               100                 105                 110
   Thr Tyr Leu Ala Tyr Asn Ala Gln Asp Met Val Phe Ala Glu Tyr Gly
           115                 120                 125
   Pro Lys Trp Lys Leu Leu Arg Lys Leu Cys Ser Leu His Met Leu Gly
       130                 135                 140
   Pro Lys Ala Leu Glu Asp Trp Ala His Val Arg Val Ser Glu Val Gly
   145                 150                 155                 160
   His Met Leu Lys Glu Met Tyr Glu Gln Ser Ser Lys Ser Val Pro Val
                   165                 170                 175
   Val Val Pro Glu Met Leu Thr Tyr Ala Met Ala Asn Met Ile Gly Arg
               180                 185                 190
   Ile Ile Leu Ser Arg Arg Pro Phe Val Ile Thr Ser Lys Leu Asp Ser
           195                 200                 205
   Ser Ala Ser Ala Ala Ser Val Ser Glu Phe Gln Tyr Met Val Met Glu
       210                 215                 220
   Leu Met Arg Met Ala Gly Leu Phe Asn Ile Gly Asp Phe Ile Pro Tyr
   225                 230                 235                 240
   Ile Ala Trp Met Asp Leu Gln Gly Ile Gln Arg Asp Met Lys Val Ile
                   245                 250                 255
   Gln Gln Lys Phe Asp Val Leu Leu Asn Lys Met Ile Lys Glu His Thr
               260                 265                 270
   Glu Ser Ala His Asp Arg Lys Asp Asn Pro Asp Phe Leu Asp Ile Leu
           275                 280                 285
   Met Ala Ala Thr Gln Glu Asn Thr Glu Gly Ile Gln Leu Asn Leu Val
       290                 295                 300
   Asn Val Lys Ala Leu Leu Leu Asp Leu Phe Thr Ala Gly Thr Asp Thr
   305                 310                 315                 320
   Ser Ser Ser Val Ile Glu Trp Ala Leu Ala Glu Met Leu Asn Asn Arg
                   325                 330                 335
   Gln Ile Leu Asn Arg Ala His Glu Glu Met Asp Gln Val Ile Gly Arg
               340                 345                 350
   Asn Arg Arg Leu Glu Gln Ser Asp Ile Pro Asn Leu Pro Tyr Phe Gln
           355                 360                 365
   Ala Ile Cys Lys Glu Thr Phe Arg Lys His Pro Ser Thr Pro Leu Asn
       370                 375                 380
   Leu Pro Arg Ile Ser Thr Glu Glu Cys Glu Val Glu Gly Phe Arg Ile
   385                 390                 395                 400
   Pro Lys Asn Thr Arg Leu Ile Val Asn Ile Trp Ala Ile Gly Arg Asp
                   405                 410                 415
   Pro Lys Val Trp Glu Asn Pro Leu Asp Phe Thr Pro Glu Arg Phe Leu
               420                 425                 430
   Ser Glu Lys His Ala Lys Ile Asp Pro Arg Gly Asn His Phe Glu Leu
           435                 440                 445
   Ile Pro Phe Gly Ala Gly Arg Arg Ile Cys Ala Gly Ala Arg Met Gly
       450                 455                 460
   Ala Ala Ser Val Glu Tyr Ile Leu Gly Thr Leu Val His Ser Phe Asp
   465                 470                 475                 480
   Trp Lys Leu Pro Asp Gly Val Val Glu Val Asn Met Glu Glu Ser Phe
                   485                 490                 495
   Gly Ile Ala Leu Gln Lys Lys Met Pro Leu Ser Ala Ile Val Thr Pro
               500                 505                 510
   Arg Leu Pro Pro Ser Ala Tyr Thr Val
           515                 520
<210> 13
   <211> 1322
   <212> DNA
   <213> Lilium sp.
<220>
   <221> CDS
   <222> (1)..(1161)
   <223> cDNA of lily DFR
<400> 13
   atg tct caa aga gga gag aga gag atg gag aat gtg gtg aaa ggc ccc        48
   Met Ser Gln Arg Gly Glu Arg Glu Met Glu Asn Val Val Lys Gly Pro
   1               5                   10                  15
   gtg gtg gtg act ggt gct agt ggc tat gtt ggt tca tgg ctt gta atg        96
   Val Val Val Thr Gly Ala Ser Gly Tyr Val Gly Ser Trp Leu Val Met
               20                  25                  30
   aag ctt ctg caa tat gga tac acg gtc cgg gct act gtg aga gac cct       144
   Lys Leu Leu Gln Tyr Gly Tyr Thr Val Arg Ala Thr Val Arg Asp Pro
           35                  40                  45
   aga gac cta aga aag acc aaa cct tta ata gat ctt cct gga gct gat       192
   Arg Asp Leu Arg Lys Thr Lys Pro Leu Ile Asp Leu Pro Gly Ala Asp
       50                  55                  60
   gaa cgg cta acc att tgg aaa gca gat ctt agc gag gat gag agc ttt       240
   Glu Arg Leu Thr Ile Trp Lys Ala Asp Leu Ser Glu Asp Glu Ser Phe
   65                  70                  75                  80
   gat gag gca atc aat ggt tgc tcc ggt gtg tac cat gtt gca act ccg       288
   Asp Glu Ala Ile Asn Gly Cys Ser Gly Val Tyr His Val Ala Thr Pro
                   85                  90                  95
   atg gat ttc gat tcc gaa gat ccg gag aat gaa gtg atc aag ccc aca       336
   Met Asp Phe Asp Ser Glu Asp Pro Glu Asn Glu Val Ile Lys Pro Thr
               100                 105                 110
   ata aat gga gta ctt ggt atc atg aaa tct tgc aaa aaa gca ggg acg      384
   Ile Asn Gly Val Leu Gly Ile Met Lys Ser Cys Lys Lys Ala Gly Thr
           115                 120                 125
   gtg aag cgt gtc att ttc act tcc tct gct gga act gtg aac gtg caa      432
   Val Lys Arg Val Ile Phe Thr Ser Ser Ala Gly Thr Val Asn Val Gln
       130                 135                 140
   gaa cat caa atg tcc gaa tat gac gag agc tcg tgg agc gac atc gac      480
   Glu His Gln Met Ser Glu Tyr Asp Glu Ser Ser Trp Ser Asp Ile Asp
   145                 150                 155                 160
   ttc tgc aga cgc gtc aag atg act gga tgg atg tac ttt gta tct aaa      528
   Phe Cys Arg Arg Val Lys Met Thr Gly Trp Met Tyr Phe Val Ser Lys
                   165                 170                 175
   acc cta gca gag aaa gct gca tgg gat ttt gca aag gag aat aat att      576
   Thr Leu Ala Glu Lys Ala Ala Trp Asp Phe Ala Lys Glu Asn Asn Ile
               180                 185                 190
   cat ttt ata agc att att cca act ttg gtg gtg ggt cct ttc atc acc      624
   His Phe Ile Ser Ile Ile Pro Thr Leu Val Val Gly Pro Phe Ile Thr
           195                 200                 205
   tca act atg cct ccc agt atg ctc aca gct ttg tca ttg atc aca gga      672
   Ser Thr Met Pro Pro Ser Met Leu Thr Ala Leu Ser Leu Ile Thr Gly
       210                 215                 220
   aat gaa gcc cac tat tcg atc tta aaa caa atc cag ctt gtt cac ttg      720
   Asn Glu Ala His Tyr Ser Ile Leu Lys Gln Ile Gln Leu Val His Leu
   225                 230                 235                 240
   gat gac gta tgc aag gcc cat att ttc ctc ttt gag aat cca gaa gcg      768
   Asp Asp Val Cys Lys Ala His Ile Phe Leu Phe Glu Asn Pro Glu Ala
                   245                 250                 255
   agt ggg aga tac ata tgc tct tct tat gat gct aca att tac gat ctt      816
   Ser Gly Arg Tyr Ile Cys Ser Ser Tyr Asp Ala Thr Ile Tyr Asp Leu
               260                 265                 270
   gca aga aag att aaa gat aga tat cct caa tat gcc atc cca caa aag      864
   Ala Arg Lys Ile Lys Asp Arg Tyr Pro Gln Tyr Ala Ile Pro Gln Lys
           275                 280                 285
   ttt gaa ggt att gat gac caa att aaa cct gtg cac ttc tct tca aag      912
   Phe Glu Gly Ile Asp Asp Gln Ile Lys Pro Val His Phe Ser Ser Lys
       290                 295                 300
   aaa ctt atg gat ctt ggt ttc aag tat cag tac act ttt gag gag atg      960
   Lys Leu Met Asp Leu Gly Phe Lys Tyr Gln Tyr Thr Phe Glu Glu Met
   305                 310                 315                 320
   ttc gat gaa gga atc cgt tca tgt atc gaa aag aaa ctg ata cca cac     1008
   Phe Asp Glu Gly Ile Arg Ser Cys Ile Glu Lys Lys Leu Ile Pro His
                   325                 330                 335
   caa aca caa gag aga tac tat gct aat gat aaa ctg aat ctg ggt tac     1056
   Gln Thr Gln Glu Arg Tyr Tyr Ala Asn Asp Lys Leu Asn Leu Gly Tyr
               340                 345                 350
   act aaa atg act aat gat aaa ctg gat ttg ggt ggc ata aag ttg aat        1104
   Thr Lys Met Thr Asn Asp Lys Leu Asp Leu Gly Gly Ile Lys Leu Asn
           355                 360                 365
   tca atg gac gag att gtg aag ggg cac aat gaa caa gtc tcc gtt gct        1152
   Ser Met Asp Glu Ile Val Lys Gly His Asn Glu Gln Val Ser Val Ala
       370                 375                 380
   ctt cag taa gcttcggagg accatacgaa agtaagccag actatgttgt                1201
   Leu Gln
   385
   agtaataact atagttgtga tttatgattc atatgtatgt gtgaggcact gagctgagac      1261
   tatgtggtag taataactat agttgtgatt tatgattcaa aaaaaaaaaa aaaaaaaaaa      1321
   a                                                                      1322
<210> 14
   <211> 386
   <212> PRT
   <213> Lilium sp.
<400> 14
   Met Ser Gln Arg Gly Glu Arg Glu Met Glu Asn Val Val Lys Gly Pro
   1               5                   10                  15
   Val Val Val Thr Gly Ala Ser Gly Tyr Val Gly Ser Trp Leu Val Met
               20                  25                  30
   Lys Leu Leu Gln Tyr Gly Tyr Thr Val Arg Ala Thr Val Arg Asp Pro
           35                  40                  45
   Arg Asp Leu Arg Lys Thr Lys Pro Leu Ile Asp Leu Pro Gly Ala Asp
       50                  55                  60
   Glu Arg Leu Thr Ile Trp Lys Ala Asp Leu Ser Glu Asp Glu Ser Phe
   65                  70                  75                  80
   Asp Glu Ala Ile Asn Gly Cys Ser Gly Val Tyr His Val Ala Thr Pro
                   85                  90                  95
   Met Asp Phe Asp Ser Glu Asp Pro Glu Asn Glu Val Ile Lys Pro Thr
               100                 105                 110
   Ile Asn Gly Val Leu Gly Ile Met Lys Ser Cys Lys Lys Ala Gly Thr
           115                 120                 125
   Val Lys Arg Val Ile Phe Thr Ser Ser Ala Gly Thr Val Asn Val Gln
       130                 135                 140
   Glu His Gln Met Ser Glu Tyr Asp Glu Ser Ser Trp Ser Asp Ile Asp
   145 150 155 160
   Phe Cys Arg Arg Val Lys Met Thr Gly Trp Met Tyr Phe Val Ser Lys
   165 170 175
   Thr Leu Ala Glu Lys Ala Ala Trp Asp Phe Ala Lys Glu Asn Asn Ile
   180 185 190
   His Phe Ile Ser Ile Ile Pro Thr Leu Val Val Gly Pro Phe Ile Thr
   195 200 205
   Ser Thr Met Pro Pro Ser Met Leu Thr Ala Leu Ser Leu Ile Thr Gly
   210 215 220
   Asn Glu Ala His Tyr Ser Ile Leu Lys Gln Ile Gln Leu Val His Leu
   225 230 235 240
   Asp Asp Val Cys Lys Ala His Ile Phe Leu Phe Glu Asn Pro Glu Ala
   245 250 255
   Ser Gly Arg Tyr Ile Cys Ser Ser Tyr Asp Ala Thr Ile Tyr Asp Leu
   260 265 270
   Ala Arg Lys Ile Lys Asp Arg Tyr Pro Gln Tyr Ala Ile Pro Gln Lys
   275 280 285
   Phe Glu Gly Ile Asp Asp Gln Ile Lys Pro Val His Phe Ser Ser Lys
   290 295 300
   Lys Leu Met Asp Leu Gly Phe Lys Tyr Gln Tyr Thr Phe Glu Glu Met
   305 310 315 320
   Phe Asp Glu Gly Ile Arg Ser Cys Ile Glu Lys Lys Leu Ile Pro His
   325 330 335
   Gln Thr Gln Glu Arg Tyr Tyr Ala Asn Asp Lys Leu Asn Leu Gly Tyr
   340 345 350
   Thr Lys Met Thr Asn Asp Lys Leu Asp Leu Gly Gly Ile Lys Leu Asn
   355 360 365
   Ser Met Asp Glu Ile Val Lys Gly His Asn Glu Gln Val Ser Val Ala
   370 375 380
   Leu Gln
   385
<210> 15
   <211> 20
   <212> DNA
   <213> Artificial Sequence
<220>
   <223> Olgonucleotide LiRG-R3
<400> 15
   tgctccgcta tcaacttatc 20
<210> 16
   <211> 1673
   <212> DNA <213> Lilium sp.
<220>
   <221> CDS
   <222> (22)..(1563)
   <223> full length of cDNA of lily F3'H
<400> 16
   aaaaccaaac cgtcagccac c atg gaa gct caa cct ctc ctc ctc ctc ctc          51
                           Met Glu Ala Gln Pro Leu Leu Leu Leu Leu
                           1               5                   10
   acc acc atc ctc atc tcc acc atc ttc atc ttc ctc cac cgc cga cgc          99
   Thr Thr Ile Leu Ile Ser Thr Ile Phe Ile Phe Leu His Arg Arg Arg
                   15                  20                  25
   cgc ggc ggc cta cca ctc cct cct ggc ccg aaa ggc tgg cct atc ctt         147
   Arg Gly Gly Leu Pro Leu Pro Pro Gly Pro Lys Gly Trp Pro Ile Leu
               30                  35                  40
   ggc aac ctt ccc caa ctc ggc ccc aaa ccc cac caa acc atg cac gcc         195
   Gly Asn Leu Pro Gln Leu Gly Pro Lys Pro His Gln Thr Met His Ala
           45                  50                  55
   cta tcc aaa ctc cac ggc ccc ctc ttc cgt ctc cgc ctc ggc tct gcc         243
   Leu Ser Lys Leu His Gly Pro Leu Phe Arg Leu Arg Leu Gly Ser Ala
       60                  65                  70
   gag gtc atc gtc gcc gcc tca gca tcg ata gca tct gag ttt ctc cgc         291
   Glu Val Ile Val Ala Ala Ser Ala Ser Ile Ala Ser Glu Phe Leu Arg
   75                  80                  85                  90
   acg cac gac aca aac ttc agc aat cgt ccc ccg aat tct ggt gcc gaa         339
   Thr His Asp Thr Asn Phe Ser Asn Arg Pro Pro Asn Ser Gly Ala Glu
                   95                  100                 105
   cac ata gct tac aac tac aat gac ctt gtc ttt gcc ccc tac ggc ccg         387
   His Ile Ala Tyr Asn Tyr Asn Asp Leu Val Phe Ala Pro Tyr Gly Pro
               110                 115                 120
   cga tgg cgt gct ctc cgc aag ctt tgc gcc ctc cac ctc ttt tcc cag         435
   Arg Trp Arg Ala Leu Arg Lys Leu Cys Ala Leu His Leu Phe Ser Gln
           125                 130                 135
   aaa gca ctc gaa gat ctc tcc tac gtc cgc gag caa gaa gtg gca ata         483
   Lys Ala Leu Glu Asp Leu Ser Tyr Val Arg Glu Gln Glu Val Ala Ile
       140                 145                 150
   ctc gca cga gac ttg gcc ggc tca tgc cgg ccg gtt aac ctc ggg gag        531
   Leu Ala Arg Asp Leu Ala Gly Ser Cys Arg Pro Val Asn Leu Gly Glu
   155                 160                 165                 170
   aca ctt aac gtt tgt gcg acg aat gcg ttg tcg agg gcg acg atc ggg        579
   Thr Leu Asn Val Cys Ala Thr Asn Ala Leu Ser Arg Ala Thr Ile Gly
                   175                 180                 185
   tgg agg gtg ttt gtg ggg aag ggg gag gat gta acg gcc gag gat ttt        627
   Trp Arg Val Phe Val Gly Lys Gly Glu Asp Val Thr Ala Glu Asp Phe
               190                 195                 200
   aag gac atg gtg atg gag gtg atg cgg ctc gcc gga gtt ttt aat att        675
   Lys Asp Met Val Met Glu Val Met Arg Leu Ala Gly Val Phe Asn Ile
           205                 210                 215
   ggt gat ttt gtg ccg ggg ctg ggt tgg ttg gat ttg cag ggg gtg gtg        723
   Gly Asp Phe Val Pro Gly Leu Gly Trp Leu Asp Leu Gln Gly Val Val
       220                 225                 230
   ggg aag atg aag aag ttg cat aag agg atg gat gct ttt ctt gat aag        771
   Gly Lys Met Lys Lys Leu His Lys Arg Met Asp Ala Phe Leu Asp Lys
   235                 240                 245                 250
   ttg ata gcg gag cac cgg acg gca gcg gcc ggc ggc gag ttc gcc gga        819
   Leu Ile Ala Glu His Arg Thr Ala Ala Ala Gly Gly Glu Phe Ala Gly
                   255                 260                 265
   aaa gat ttg ttg agt gtg atg gtt agg ttg cag gag gag gga gag ggg        867
   Lys Asp Leu Leu Ser Val Met Val Arg Leu Gln Glu Glu Gly Glu Gly
               270                 275                 280
   gag ggt ggg aaa ctg acc gac act gaa att aag gcg ctg tta ctg aac        915
   Glu Gly Gly Lys Leu Thr Asp Thr Glu Ile Lys Ala Leu Leu Leu Asn
           285                 290                 295
   ttg ttc aca gca ggg aca gac acc acc tcc agc acc gta gag tgg gct        963
   Leu Phe Thr Ala Gly Thr Asp Thr Thr Ser Ser Thr Val Glu Trp Ala
       300                 305                 310
   ctg gcc gag ctc atc cgt cac cct cca ctc ctc cac caa gct cag aaa       1011
   Leu Ala Glu Leu Ile Arg His Pro Pro Leu Leu His Gln Ala Gln Lys
   315                 320                 325                 330
   gag ctt gat act atc gtc ggt cac gac cgt ctc gtc tct gaa tcc gac       1059
   Glu Leu Asp Thr Ile Val Gly His Asp Arg Leu Val Ser Glu Ser Asp
                   335                 340                 345
   ctc ccc cac ctc ccc ttc cta agt gcc atc atc aaa gaa act ttc cga       1107
   Leu Pro His Leu Pro Phe Leu Ser Ala Ile Ile Lys Glu Thr Phe Arg
               350                 355                 360
   ctc cac cca tcc acc cca ctc tca ctc cca cgg atg gcc tcc gaa tca       1155
   Leu His Pro Ser Thr Pro Leu Ser Leu Pro Arg Met Ala Ser Glu Ser
           365                 370                 375
   tgc acc atc aat ggc tac caa att cct aaa aac gcc act cta ctg gta       1203
   Cys Thr Ile Asn Gly Tyr Gln Ile Pro Lys Asn Ala Thr Leu Leu Val
       380                 385                 390
   aat gtg tgg gct atc gca cgt gac cca gct gtt tgg acg gcc ccg gta       1251
   Asn Val Trp Ala Ile Ala Arg Asp Pro Ala Val Trp Thr Ala Pro Val
   395                 400                 405                 410
   gag ttc aaa ccg gac agg ttc atg ccg ggt ggg gtt ggt gct cac ttg       1299
   Glu Phe Lys Pro Asp Arg Phe Met Pro Gly Gly Val Gly Ala His Leu
                   415                 420                 425
   gat gta aag ggt agc gac ttc gag atg atc cct ttt gga gcc ggg agg       1347
   Asp Val Lys Gly Ser Asp Phe Glu Met Ile Pro Phe Gly Ala Gly Arg
               430                 435                 440
   agg att tgc gct ggt atg agc ttg ggg ctg agg atg gtg acg ttc atg       1395
   Arg Ile Cys Ala Gly Met Ser Leu Gly Leu Arg Met Val Thr Phe Met
           445                 450                 455
   aca gcg aca cta gtg cat ggg ttc gaa tgg aag ctc ccg aat ggg gtg       1443
   Thr Ala Thr Leu Val His Gly Phe Glu Trp Lys Leu Pro Asn Gly Val
       460                 465                 470
   gtg gct gag aag ttg gac atg gag gag gct tac ggt ctg acc cta cag       1491
   Val Ala Glu Lys Leu Asp Met Glu Glu Ala Tyr Gly Leu Thr Leu Gln
   475                 480                 485                 490
   agg gcg gta ccg ctg atg gtg gtg cca gtg ccg agg ctc gcg aag cag       1539
   Arg Ala Val Pro Leu Met Val Val Pro Val Pro Arg Leu Ala Lys Gln
                   495                 500                 505
   gcg tat ggg aag aat gag aaa ctc tagtgtttgc atcttaataa gaaaaactta      1593
   Ala Tyr Gly Lys Asn Glu Lys Leu
               510
   ttcgccatga ttatcatgtt ataataatga cacttagaat taataaaaat aaatttaaaa     1653
   tcaaaaaaaa aaaaaaaaaa                                                 1673
<210> 17
   <211> 514 <212> PRT
   <213> Lilium sp.
<400> 17
   Met Glu Ala Gln Pro Leu Leu Leu Leu Leu Thr Thr Ile Leu Ile Ser
   1               5                   10                  15
   Thr Ile Phe Ile Phe Leu His Arg Arg Arg Arg Gly Gly Leu Pro Leu
               20                  25                  30
   Pro Pro Gly Pro Lys Gly Trp Pro Ile Leu Gly Asn Leu Pro Gln Leu
           35                  40                  45
   Gly Pro Lys Pro His Gln Thr Met His Ala Leu Ser Lys Leu His Gly
       50                  55                  60
   Pro Leu Phe Arg Leu Arg Leu Gly Ser Ala Glu Val Ile Val Ala Ala
   65                  70                  75                  80
   Ser Ala Ser Ile Ala Ser Glu Phe Leu Arg Thr His Asp Thr Asn Phe
                   85                  90                  95
   Ser Asn Arg Pro Pro Asn Ser Gly Ala Glu His Ile Ala Tyr Asn Tyr
               100                 105                 110
   Asn Asp Leu Val Phe Ala Pro Tyr Gly Pro Arg Trp Arg Ala Leu Arg
           115                 120                 125
   Lys Leu Cys Ala Leu His Leu Phe Ser Gln Lys Ala Leu Glu Asp Leu
       130                 135                 140
   Ser Tyr Val Arg Glu Gln Glu Val Ala Ile Leu Ala Arg Asp Leu Ala
   145                 150                 155                 160
   Gly Ser Cys Arg Pro Val Asn Leu Gly Glu Thr Leu Asn Val Cys Ala
                   165                 170                 175
   Thr Asn Ala Leu Ser Arg Ala Thr Ile Gly Trp Arg Val Phe Val Gly
               180                 185                 190
   Lys Gly Glu Asp Val Thr Ala Glu Asp Phe Lys Asp Met Val Met Glu
           195                 200                 205
   Val Met Arg Leu Ala Gly Val Phe Asn Ile Gly Asp Phe Val Pro Gly
       210                 215                 220
   Leu Gly Trp Leu Asp Leu Gln Gly Val Val Gly Lys Met Lys Lys Leu
   225                 230                 235                 240
   His Lys Arg Met Asp Ala Phe Leu Asp Lys Leu Ile Ala Glu His Arg
                   245                 250                 255
   Thr Ala Ala Ala Gly Gly Glu Phe Ala Gly Lys Asp Leu Leu Ser Val
               260                 265                 270
   Met Val Arg Leu Gln Glu Glu Gly Glu Gly Glu Gly Gly Lys Leu Thr
           275                 280                 285
   Asp Thr Glu Ile Lys Ala Leu Leu Leu Asn Leu Phe Thr Ala Gly Thr
       290                 295                 300
   Asp Thr Thr Ser Ser Thr Val Glu Trp Ala Leu Ala Glu Leu Ile Arg
   305                 310                 315                 320
   His Pro Pro Leu Leu His Gln Ala Gln Lys Glu Leu Asp Thr Ile Val
                   325                 330                 335
   Gly His Asp Arg Leu Val Ser Glu Ser Asp Leu Pro His Leu Pro Phe
               340                 345                 350
   Leu Ser Ala Ile Ile Lys Glu Thr Phe Arg Leu His Pro Ser Thr Pro
           355                 360                 365
   Leu Ser Leu Pro Arg Met Ala Ser Glu Ser Cys Thr Ile Asn Gly Tyr
       370                 375                 380
   Gln Ile Pro Lys Asn Ala Thr Leu Leu Val Asn Val Trp Ala Ile Ala
   385                 390                 395                 400
   Arg Asp Pro Ala Val Trp Thr Ala Pro Val Glu Phe Lys Pro Asp Arg
                   405                 410                 415
   Phe Met Pro Gly Gly Val Gly Ala His Leu Asp Val Lys Gly Ser Asp
               420                 425                 430
   Phe Glu Met Ile Pro Phe Gly Ala Gly Arg Arg Ile Cys Ala Gly Met
           435                 440                 445
   Ser Leu Gly Leu Arg Met Val Thr Phe Met Thr Ala Thr Leu Val His
       450                 455                 460
   Gly Phe Glu Trp Lys Leu Pro Asn Gly Val Val Ala Glu Lys Leu Asp
   465                 470                 475                 480
   Met Glu Glu Ala Tyr Gly Leu Thr Leu Gln Arg Ala Val Pro Leu Met
                   485                 490                 495
   Val Val Pro Val Pro Arg Leu Ala Lys Gln Ala Tyr Gly Lys Asn Glu
               500                 505                 510
   Lys Leu
<210> 18
   <211> 34
   <212> DNA
   <213> Artificial Sequence
<220>
   <223> primer cLD2L
<400> 18
   agggtctctc acagtagccc gaaccgtgta gcca 34
<210> 19
   <211> 34
   <212> DNA
   <213> Artificial Sequence
<220>
   <223> primer LD1L
<400> 19
   actagatctt cctggagctg atgaacggct aacc 34
<210> 20
   <211> 35
   <212> DNA
   <213> Artificial Sequence
<220>
   <223> primer cLD1L
<400> 20
   aacatagcca ctagctccag taaccaccac gggtc 35
<210> 21
   <211> 35
   <212> DNA
   <213> Artificial Sequence
<220>
   <223> primer LD3L
<400> 21
   agcaaatcca gcttgttcac ttggatgacg tatgc 35
<210> 22
   <211> 36
   <212> DNA
   <213> Artificial Sequence
<220>
   <223> oligonucleotide AscI-HindIII-LDE6-F
<400> 22
   ggcgcgccaa gcttgaattc ttagcggaaa ttatcc 36
<210> 23
   <211> 25
   <212> DNA
   <213> Artificial Sequence
<220>
   <223> oligonucleotide XbaI-LDE6-R
<400> 23
   tctagaatct ctctctctcc ccctc 25
<210> 24
   <211> 613
   <212> DNA
   <213> Lilium sp.
<220>
   <221> promoter
   <222> (23)..(590)
   <223> lily DFR promoter
<400> 24
   gaattcttag cggaaattat ccggatccgt ccgaaacata agataatccc cgaacctgat      60
   cttttcgaga tttggactct ggacttctgt caagaatgcg atttcagata cagtattact     120
   cccatccaat ttgcttgtag agattttgat cttttatgtg ttttgcataa gtcttccttt     180
   aatgaggctt ccttccttga aaatattcct accaatcaac atatgacgcg gccgtctgtg     240
   ctcctctcat ggtacgagaa acatgggttc ttttaagtga ggaaaacgaa aacggtaggc     300
   cagagtccaa ttccaacaac caatcaaacc agttgcatgt cggaacgtgg ttcattaccc     360
   agttaacctt ctaccattct ctaggctgaa agttagatac caagttccat tgtgctacgt     420
   accacactca atctaaaata agagattaat atacatataa cagctgaaaa ttaaataccc     480
   actttccatt ttgccaccta ccacgctcaa cctaacatga cagattcata tatctagcat     540
   ccgagattca ttatataaca gtcctacgtt tcaaagagaa gagagagaga gggggagaga     600
   gagagattct aga                                                        613